(19) Europäisches Patentamt
European Patent Office
Office européen des brevets

(11) Veröffentlichungsnummer: **0 272 628 B1**

(12) **EUROPÄISCHE PATENTSCHRIFT**

(45) Veröffentlichungstag der Patentschrift: **29.04.92**

(51) Int. Cl.5: **C07D 487/04**, A01N 43/90, //(C07D487/04,251:00,249:00)

(21) Anmeldenummer: **87118740.7**

(22) Anmeldetag: **17.12.87**

Die Akte enthält technische Angaben, die nach dem Eingang der Anmeldung eingereicht wurden und die nicht in dieser Patentschrift enthalten sind.

# (54) 6,7-Dihydro-[1,2,4]triazolo[1,5-a][1,3,5]triazin-2-sulfonsäureamide, Verfahren zu ihrer Herstellung und ihre Verwendung als Mittel mit herbizider und wachstumsregulierender Wirkung.

(30) Priorität: **19.12.86 DE 3644343**

(43) Veröffentlichungstag der Anmeldung:
**29.06.88 Patentblatt 88/26**

(45) Bekanntmachung des Hinweises auf die Patenterteilung:
**29.04.92 Patentblatt 92/18**

(84) Benannte Vertragsstaaten:
**AT BE CH DE ES FR GB GR IT LI LU NL SE**

(56) Entgegenhaltungen:
**EP-A- 0 142 152**
**EP-A- 0 150 974**
**DD-A- 205 905**
**DE-A- 2 236 340**
**DE-A- 2 720 792**
**US-A- 4 685 958**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Westermann, Jürgen, Dr.**
**Elberfelderstrasse 19**
**W-1000 Berlin 21(DE)**
Erfinder: **Arndt, Friedrich, Dr.**
**Mühlenfeldstrasse 10**
**W-1000 Berlin 28(DE)**
Erfinder: **Krüger, Martin, Dr.**
**Schluchseestrasse 65**
**W-1000 Berlin 28(DE)**
Erfinder: **Rees, Richard, Dr.**
**Speerweg 8**
**W-1000 Berlin 28(DE)**
Erfinder: **Kötter, Clemens, Dr.**
**Laurinsteig 26**
**W-1000 Berlin 28(DE)**

EP 0 272 628 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 6,7-Dihydro-[1,2,4]triazolo[1,5-a][1,3,5]triazin-2-sulfonsäureamide, Verfahren zu ihrer Herstellung und ihre Verwendung als Mittel mit herbizider und wachstumsregulierender Wirkung.

Es ist bereits bekannt, daß Triazolo-pyrimidin-sulfonamide eine herbizide Wirkung besitzen (EP-Anmeldungen 0 142 152 und 0 150 974). Es ist ferner bekannt, daß Triazolo-triazin-Derivate herbizide Eigenschaften haben (DE 2720792 und DD 205 905). Häufig ist jedoch die Herbizidwirkung der bekannten Verbindungen nicht ausreichend, beziehungsweise es treten bei entsprechender Herbizidwirkung Selektivitätsprobleme in landwirtschaftlichen Hauptkulturen auf.

Aufgabe der vorliegenden Erfindung ist die Bereitstellung von neuen Verbindunggen, die diese Nachteile nicht aufweisen und in ihren biologischen Eigenschaften den bisher bekannten Verbindungen überlegen sind.

Es wurde nun gefunden, daß 6,7-Dihydro-[1,2,4]triazolo[1,5-a][1,3,5]triazin-2-sulfonsäureamide der allgemeinen Formel I

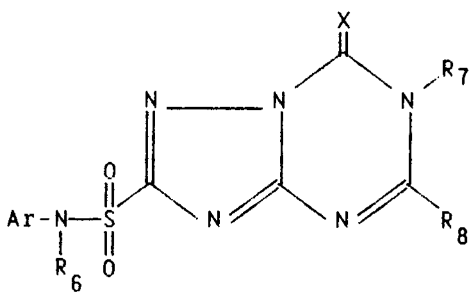

(I) ,

in der

Ar eine Phenylgruppe der allgemeinen Formel

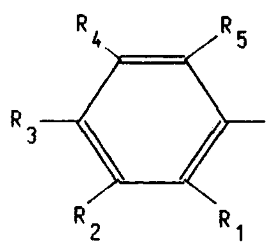

,

$R_1$, $R_2$, $R_3$, $R_4$ und $R_5$, die gleich oder verschieden sind, ein Wasserstoffatom, einen gegebenenfalls ein- oder mehrfach, gleich oder verschieden durch Halogen, Hydroxy, Halogen-$C_1$-$C_4$-alkyl, $C_1$-$C_4$-Alkoxy, Halogen-$C_1$-$C_4$-alkoxy, $C_1$-$C_4$-Alkylthio, $C_1$-$C_4$-Alkylsulfinyl oder $C_1$-$C_4$-Alkylsulfonyl substituierten $C_1$-$C_6$-Alkyl-, $C_2$-$C_6$-Alkenyl- oder $C_2$-$C_6$-Alkinylrest, ein Halogenatom, eine $C_1$-$C_4$-Alkoxygruppe,
eine Aminocarbonylgruppe

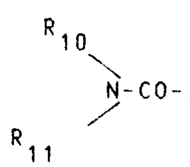

eine Aminogruppe

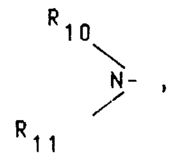

eine Cyanogruppe, eine Nitrogruppe, eine Schwefel enthaltende Gruppe $R_9$-$S(O)_n$-, eine Acylgruppe $R_9$-CO-, eine Gruppe $R_9$-O-CO-$(CH_2)_n$- oder einen durch $C_1$-$C_4$-Alkyl, Halogen oder Nitro substituierten Phenyl- oder Phenoxyrest,

$R_6$ ein Wasserstoffatom, eine Acylgruppe $R_9$-CO-, eine Gruppe $R_9$-O-CO-, eine $C_1$-$C_6$-Alkylgruppe, eine $C_2$-$C_6$-Alkenylgruppe, eine $C_2$-$C_6$-Alkinylgruppe, eine Phenyl-$C_1$-$C_4$-alkylgruppe, eine Aminocarbonylgruppe

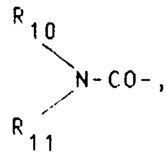

ein Alkalimetallatom, ein einwertiges Metalläquivalent aus der Gruppe der Erdalkalimetalle und weiterer Metalle oder einen gegebenenfalls durch $C_1$-$C_6$-Alkyl substituierten Ammoniumrest,

$R_7$ und $R_8$, die gleich oder verschieden sind, ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl oder Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest, einen durch $R_1$, $R_2$, $R_3$, $R_4$ und $R_5$ substituierten Phenylrest, eine Acylgruppe $R_9$-CO-, eine Gruppe $R_9$-O-CO-$(CH_2)_n$-, eine Aminocarbonylgruppe

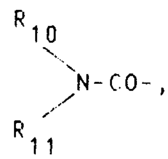

oder eine Sulfonylgruppe $R_9$-$SO_2$-,

$R_9$ ein Wasserstoffatom, eine gegebenenfalls ein- oder mehrfach, gleich oder verschieden durch Halogen, Hydroxy oder $C_1$-$C_4$-Alkoxy substituierte $C_1$-$C_6$-Alkyl-, $C_2$-$C_6$-Alkenyl-, $C_2$-$C_6$-Alkinyl- oder Phenyl-$C_1$-$C_4$-alkylgruppe oder eine gegebenenfalls durch Halogen, Nitro oder $C_1$-$C_4$-Alkyl substituierte Phenylgruppe,

$R_{10}$ und $R_{11}$, die gleich oder verschieden sind, ein Wasserstoffatom, einen gegebenenfalls ein- oder mehrfach, gleich oder verschieden durch Halogen, Hydroxy oder $C_1$-$C_4$-Alkoxy substituierten $C_1$-$C_6$-Alkyl-, $C_2$-$C_6$-Alkenyl- oder $C_2$-$C_6$-Alkinylrest oder

$R_{10}$ und $R_{11}$ gemeinsam mit dem benachbarten Stickstoffatom die Pyrrolidinyl-, Piperidino- oder Morpholinogruppe und

X ein Sauerstoff- oder Schwefelatom

n 0, 1 oder 2

bedeuten, eine interessante herbizide und wachstumsregulierende Wirkung zeigen.

Der Begriff "Halogen" im Zusammenhang mit Alkyl, Alkenyl, Alkinyl oder Phenyl bedeutet, daß ein oder mehrere Wasserstoffatome durch ein oder mehrere Halogenatome ersetzt sind.

Die Bezeichnung "Halogen" umfaßt Fluor, Chlor, Brom und Jod.

Als besonders wirksam haben sich solche 6,7-Dihydro-[1,2,4]triazolo[1,5-a][1,3,5]triazin-2-sulfonsäureamide der allgemeinen Formel I erwiesen, bei denen Ar eine Phenylgruppe der allgemeinen Formel

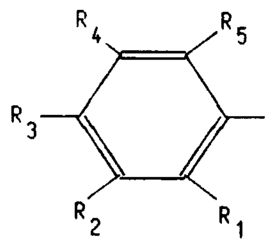

| | |
|---|---|
| $R_1$ und $R_5$, | die gleich oder verschieden sind, ein Halogenatom, eine Methylgruppe, eine Trifluormethylgruppe, eine Nitrogruppe, eine Methoxygruppe oder eine Methoxycarbonylgruppe, |
| $R_2$, $R_3$ und $R_4$, | die gleich oder verschieden sind, ein Wasserstofatom, ein Halogenatom, eine Trifluormethyl- oder eine $C_1$-$C_4$-Alkylgruppe, |
| $R_6$ | ein Wasserstoffatom, ein einwertiges Metalläquivalent oder eine $C_1$-$C_4$-Acylgruppe, |
| $R_7$ und $R_8$, | die gleich oder verschieden sind, ein Wasserstoffatom, eine $C_1$-$C_4$-Acylgruppe, eine $C_1$-$C_4$-Alkylgruppe oder eine $C_2$-$C_4$-Alkenylgruppe oder eine Phenylgruppe und |
| X | ein Sauerstoff- oder Schwefelatom |

bedeuten.

Die erfindungsgemäßen Verbindungen der allgemeinen Formel I können zum Beispiel hergestellt werden, indem man

A) Amine der allgemeinen Formel II,

Ar-NH-$R_6$ (II) ,

in der Ar die oben angegebene Bedeutung hat und $R_6$ ein Wasserstoffatom, eine $C_1$-$C_6$-Alkyl-, $C_2$-$C_6$-Alkenyl- oder $C_2$-$C_6$-Alkinylgruppe bedeutet, mit einem Sulfonsäurechlorid der allgemeinen Formel III

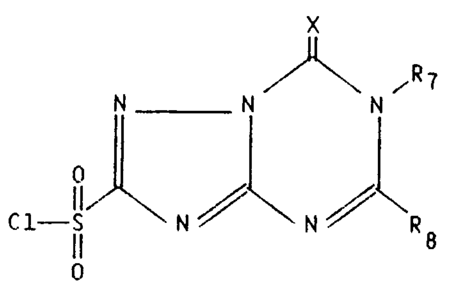

(III) ,

in der $R_7$, $R_8$ und X die oben angegebenen Bedeutungen haben, in einem geeigneten Lösungsmittel in Gegenwart eines Säureakzeptors umsetzt, oder

B) eine Verbindung der allgemeinen Formel IX

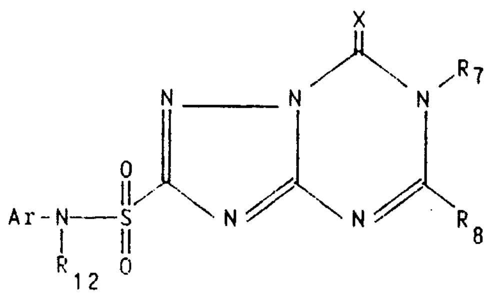

(IX) ,

in der Ar, $R_7$, $R_8$ und X die oben angegebenen Bedeutungen haben und $R_{12}$ ein Wasserstoffatom oder ein einwertiges Metalläquivalent bedeutet, mit Verbindungen der allgemeinen Formel X

$R_6$-Hal (X) ,

in der $R_6$ die oben genannte Bedeutungen hat aber nicht für ein Wasserstoffatom steht, und Hal Chlor oder Brom bedeutet, oder der allgemeinen Formel XI

$R_9$-CO-Hal (XI) ,

in der $R_9$ die oben genannte Bedeutung hat, aber nicht für ein Wasserstoffatom steht, und Hal Chlor oder Brom bedeutet, oder der allgemeinen Formel XII

$R_9$-CO-O-CO-$R_9$ (XII) ,

in der $R_9$ die oben genannte Bedeutung hat, in einem geeigneten Lösungsmittel umsetzt, oder
C) eine Verbindung der allgemeinen Formel XIII

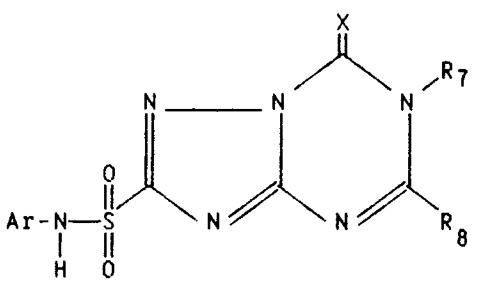

(XIII) ,

in der Ar, $R_7$, $R_8$ und X die oben angegebenen Bedeutungen haben, mit einer Verbindung der allgemeinen Formel XIV

M - Y (XIV) ,

in der M ein einwertiges Metalläquivalent und Y ein Wasserstoffatom, eine Hydroxy-, Niederalkyl-, Niederalkoxy- oder Aminogruppe bedeutet, in einem Lösungsmittel umsetzt, oder

D) eine Verbindung der allgemeinen Formel IV

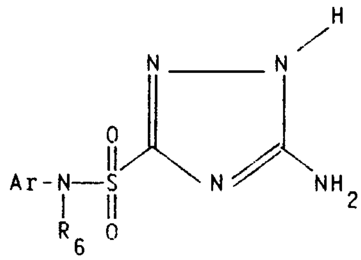

(IV) ,

in der Ar die oben angegebene Bedeutung hat und $R_6$ ein Wasserstoffatom oder eine $C_1$-$C_6$-Alkyl-, $C_2$-$C_6$-Alkenyl- oder $C_2$-$C_6$-Alkinylgruppe bedeutet, mit einem Isocyanat oder Isothiocyanat der Formel V

$R_7$-NCX (V) ,

in der $R_7$ und X die oben angegebenen Bedeutungen haben, in einem geeigneten Lösungsmittel gegebenenfalls in Gegenwart eines Säureakzeptors beziehungsweise Katalysators umsetzt, und die entstandenen Verbindungen der allgemeinen Formel VI

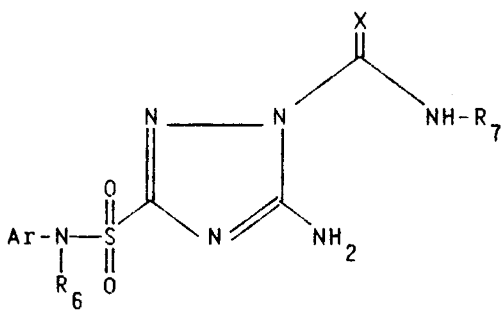

(VI) ,

in der Ar, $R_7$ und X die oben angegebenen Bedeutungen haben und $R_6$ ein Wasserstoffatom, eine $C_1$-$C_6$-Alkyl-, $C_2$-$C_6$-Alkenyl- oder $C_2$-$C_6$-Alkinylgruppe bedeutet, mit einem Orthoester der allgemeinen Formel VII

$R_8$-$C(OR_9)_3$ (VII) ,

in der $R_9$ die oben angegebene Bedeutung hat, aber nicht für ein Wasserstoffatom steht, und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest oder einen durch $R_1$, $R_2$, $R_3$, $R_4$ und $R_5$ substituierten Phenylrest bedeutet, in einem geeigneten Lösungsmittel, das auch der Orthoester selbst sein kann, umsetzt, oder

E) eine Verbindung der allgemeinen Formel IV

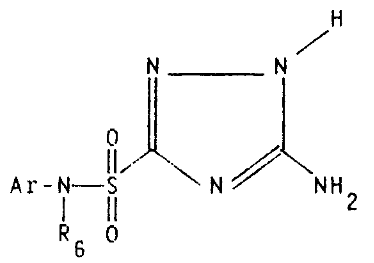

(IV) ,

in der Ar die oben angegebene Bedeutung hat und $R_6$ ein Wasserstoffatom oder eine $C_1$-$C_6$-Alkyl-, $C_2$-$C_6$-Alkenyl- oder $C_2$-$C_6$-Alkinylgruppe bedeutet, mit einem Orthoester der allgemeinen Formel VII

$R_8$-C($OR_9$)$_3$ (VIII) ,

in der $R_9$ die oben angegebene Bedeutung hat, aber nicht für ein Wasserstoffatom steht, und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl-oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest oder einen durch $R_1$, $R_2$, $R_3$, $R_4$ und $R_5$ substituierten Phenylrest bedeutet, zu Verbindungen der allgemeinen Formel VIII

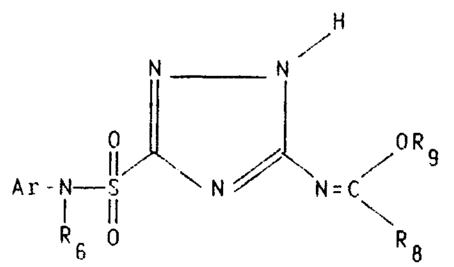

(VIII) ,

in der Ar und $R_9$ die oben angegebenen Bedeutungen haben, $R_9$ aber nicht für ein Wasserstoffatom steht, $R_6$ ein Wasserstoffatom oder eine $C_1$-$C_6$-Alkyl-, $C_2$-$C_6$-Alkenyl- oder $C_2$-$C_6$-Alkinylgruppe und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest oder einen durch $R_1$, $R_2$, $R_3$, $R_4$ und $R_5$ substituierten Phenylrest bedeuten, in einem geeigneten Lösungsmittel, das auch der Orthoester selbst sein kann, umsetzt und dann die Verbindungen der allgemeinen Formel VIII mit einem Isocyanat oder Isothiocyanat der Formel V

$R_7$-NCX (V)

in der $R_7$ und X die oben angegebenen Bedeutungen haben, in einem geeigneten Lösungsmittel gegebenenfalls in Gegenwart eines Säureakzeptors beziehungsweise Katalysators umsetzt.

Die einzelnen Verfahrensvarianten werden vorzugsweise in Gegenwart eines Verdünnungsmittels durchgeführt. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum

Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfone und Sulfoxide, wie zum Beispiel Dimethylsulfoxid und Sulfolan, und Basen, wie zum Beispiel Pyridin.

Die Reaktionen werden vorzugsweise zwischen Raumtemperatur und dem Siedepunkt des jeweiligen Reaktionsgemisches durchgeführt. Die Reaktionen lassen sich bei dem Druck der Umgebung durchführen, wenngleich sie auch bei erhöhtem beziehungsweise vermindertem Druck durchgeführt werden können.

Die Verfahrensvariante A) wird vorzugsweise in chlorierten Kohlenwasserstoffen wie Dichlormethan oder Dichlorethan, in Anwesenheit eines Katalysators beziehungsweise Säureakzeptors durchgeführt. Beispiele hierfür sind tertiäre Amine, wie zum Beispiel Triethylamin, Diisopropylethylamin, N-Methylmorpholin, 4-Dimethylaminopyridin und Pyridin. Pyridin kann sowohl als Katalysator als auch als Lösungsmittel bei dieser Reaktion verwendet werden.

Die Verfahrensvarianten D) und E) werden bevorzugt in Gegenwart von Verdünnungsmitteln wie aliphatischen, alicyclischen und aromatischen Kohlenwasserstoffen, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ethern, wie zum Beispiel Diisopropylether, Dibutylether, Propylenoxid, Dioxan und Tetrahydrofuran, Ketonen, wie zum Beispiel Aceton, Methylethylketon, Methylisopropylketon und Methylisobutylketon, Nitrilen, wie zum Beispiel Acetonitril und Propionitril, Estern, wie zum Beispiel Ethylacetat und Amylacetat, Säureamiden, wie zum Beispiel Dimethylformamid und Dimethylacetamid, Sulfonen und Sulfoxiden, wie zum Beispiel Dimethylsulfoxid und Sulfolan, und Basen, wie zum Beispiel Pyridin, und gegebenenfalls in Anwesenheit eines Katalysators beziehungsweise Säureakzeptors durchgeführt. Beispiele hierfür sind tertiäre Amine, wie zum Beispiel Triethylamin, Diisopropylethylamin, N-Methylmorpholin, 4-Dimethylaminopyridin oder Pyridin.

Die Verfahrensvariante B) wird vorzugsweise so durchgeführt, daß man eine Verbindung der allgemeinen Formel IX in einem geeigneten Lösungsmittel mit Verbindungen der allgemeinen Formeln X, XI oder XII umsetzt, die in der Regel schwer löslichen anorganischen Salze abfiltriert und die gewünschten Verbindungen nach Abdampfen der Lösungsmittel erhält.

Die Verfahrensvariante C) wird vorzugsweise so durchgeführt, daß man eine Verbindung der allgemeinen Formel XIII in einem geeigneten Lösungsmittel mit einer Metallbase, wie einem Metallhydroxid, Metallhydrid, Metallalkyl oder Metallamid, umsetzt und die in der Regel schwerer löslichen Salze abfiltriert oder nach Abdampfen der Lösungsmittel erhält.

Die nach den oben genannten Verfahren hergestellten erfindungsgemäßen Verbindungen können nach den üblichen Verfahren aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion.

Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel farb- und geruchlose Kristalle dar, die wenig löslich in Wasser und in aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff, aromatischen Kohlenwasserstoffen, wie Benzol, Toluol und Xylol, Ethern, wie Diethylether, Tetrahydrofuran und Dioxan, Carbonsäurenitrilen, wie Acetonitril, Alkoholen, wie Methanol und Ethanol, Carbonsäureamiden, wie Dimethylformamid, und Sulfoxiden, wie Dimethylsulfoxid, sind.

Die Sulfonsäurechloride der allgemeinen Formel III sind neu und lassen sich aus in der Literatur beschriebenen beziehungsweise nach bekannten Verfahren darstellbaren 2-Benzylthio-6,7-dihydro-[1,2,4]-triazolo[1,5-a][1,3,5]triazin-7-onen der allgemeinen Formel XV

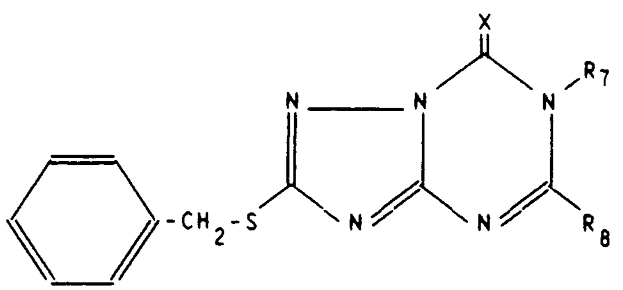

(XV) ,

in der $R_7$, $R_8$ und X die oben angegebenen Bedeutungen haben, durch Umsetzung mit Chlor in Wasser oder Wasser/Essigsäuregemischen herstellen.

Amine der allgemeinen Formel II, Isocyanate beziehungsweise Isothiocyanate der allgemeinen Formel V und Orthoester der allgemeinen Formel VII sind zum Teil käuflich oder nach bekannten beziehungsweise in der Literatur beschriebenen Verfahren darstellbar.

Die erfindungsgemäßen Wirkstoffe beeinflussen das Pflanzenwachstum und können deshalb als Wachstumsregulatoren und insbesondere als Umkrautbekämpfungsmittel verwendet werden. Überraschenderweise zeigen die erfindungsgemäßen Verbindungen eine breite Wirkung gegen monokotyle und dikotyle Unkräuter bei guter Verträglichkeit gegenüber Kulturpflanzen. Ob die erfindungsgemäßen Verbindungen als totale oder selektive Herbizide wirken, hängt im wesentlichen von der Aufwandmenge, aber auch von der Anwendungsart und dem Anwendungszeitpunkt ab.

Die Anwendung kann im Vorsaat-, Vorauflauf- oder Nachauflaufverfahren erfolgen.

Die erfindungsgemäßen Wirkstoffe können zum Beispiel gegen die folgenden Pflanzengattungen eingesetzt werden:
Dikotyle Unkräuter der Gattungen Polygonum, Sinapis, Atriplex, Spergula, Stellaria, Galium, Viola, Cirsium, Amaranthus, Ipomoea, Xanthium, Abutilon, Chenopodium, Cassia, Convolvulus, Mentha, Veronica, Matricaria, Solanum, Lamium, Thlaspi, Capsella, Datura, Galinsoga, Mercurialis, Rhaphanus, Vicia, Portulaca, Physalis, Sida, Anoda, Euphorbia, Myosotis, Centaurea, Brassica, Chrysanthemum und Helianthus.

Monokotyle Unkräuter der Gattungen Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Agrostis, Alopecurus, Apera, Rottboellia, Triticum und Hordeum.

Die Verbindungen können in vielen landwirtschaftlichen Hauptkulturen, wie zum Beispiel Weizen, Gerste, Reis und Soja, angewendet werden.

Die Verwendung der erfindungsgemäßen Wirkstoffe ist keineswegs auf die genannten Unkrautgattungen und Kulturpflanzen beschränkt, sondern erstreckt sich in gleicher Weise auf andere Pflanzen.

Die Verbindungen eignen sich auch zur Unkrautbekämpfung auf Industrie-und Gleisanlagen, auf Wegen und Plätzen mit und ohne Baumbewuchs, in Forst-, Ziergehölz-, Beerenfrucht- und Hopfenanlagen sowie in Plantagenkulturen.

Die angewandte Wirkstoffmenge kann in einem größeren Bereich schwanken. Sie hängt im wesentlichen von der Art des gewünschten Effekts ab. Im allgemeinen liegen die Aufwandmengen zwischen 0,01 und 1 kg Wirkstoff pro Hektar Bodenfläche, vorzugsweise zwischen 0,1 und 0,5 kg Wirkstoff pro Hektar.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden. Beispielsweise eignen sich als herbizid wirksame Mischungspartner diejenigen Wirkstoffe, die in Weed Abstracts, Vol. 35, No.5, 1986, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acylphosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier-und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen varrieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.)

| | |
|---|---|
| 25 Gewichtsprozent | Wirkstoff |
| 60 Gewichtsprozent | Kaolin |
| 10 Gewichtsprozent | Kieselsäure |
| 5 Gewichtsprozent | einer Mischung aus dem Calciumsalz der Ligninsulfonsäure und dem Natriumsalz des N-Methyl-N-oleyl-taurins |

2.)

| | |
|---|---|
| 40 Gewichtsprozent | Wirkstoff |
| 25 Gewichtsprozent | Tonmineralien |
| 25 Gewichtsprozent | Kieselsäure |
| 10 Gewichtsprozent | einer Mischung aus dem Calciumsalz der Ligninsulfonsäure und Alkylphenylpolyglycolethern |

B) **Paste**

| | |
|---|---|
| 45 Gewichtsprozent | Wirkstoff |
| 5 Gewichtsprozent | Natriumaluminiumsilikat |
| 15 Gewichtsprozent | Cetylpolyglycolether mit 8 Mol Ethylenoxid |
| 2 Gewichtsprozent | Spindelöl |
| 10 Gewichtsprozent | Polyethylenglycol |
| 23 Gewichtsprozent | Wasser |

C) **Emulsionskonzentrat**

| | |
|---|---|
| 25 Gewichtsprozent | Wirkstoff |
| 15 Gewichtsprozent | Cyclohexanon |
| 55 Gewichtsprozent | Xylol |
| 5 Gewichtsprozent | einer Mischung aus dem Calciumsalz der Dodecylbenzolsulfonsäure und Nonylphenylpolyoxyethylen |

Die nachstehenden Beispiele beschreiben die Herstellung der erfindungsgemäßen Verbindungen:

**Beispiel 1**

5,6-Dimethyl-7-oxo-6,7-dihydro-[1,2,4]triazolo[1,5-a][1,3,5]triazin-2-sulfonsäure-(N-methyl-2,6-dichloranilid)

(nach Verfahrensvariante D))

1.30 g (3,4 mmol) 5-Amino-1-methylaminocarbonyl-1,2,4-triazol-3-sulfonsäure-(N-methyl-2,6-dichloranilid) werden in 10 ml Orthoessigsäuretriethylester mit 5 Tropfen Eisessig 12 Stunden bei 120 °C gerührt. Man filtriert die unlöslichen Rückstände ab und engt ein. Das Produkt wird mit Hexan/Essigester-Gemisch über Kieselgel chromatographiert.

Ausbeute: 0,77 g = 56 % der Theorie
Fp.: 286-288 °C

Elementaranalyse

| | |
|---|---|
| ber. (%): | C 38,72 H 3,00 N 20,84 S 7.95 Cl 17,58 |
| gef. (%): | C 38.54 H 3,10 N 20,79 S 8,01 Cl 17,59 |

Herstellung der Ausgangsverbindungen zu Beispiel 1

a) 5-Amino-1-methylaminocarbonyl-1,2,4-triazol-3-sulfonsäure-(N-methyl-2,6-dichloranilid)

2,0 g (6,2 mmol) 5-Amino-1,2,4-triazol-3-sulfonsäure-(N-methyl-2,6-dichloranilid) werden in 30 ml Tetrahydrofuran suspendiert und mit 0,5 ml (8,5 mmol) Methylisocyanat und 0,9 ml (6,5 mmol) Triethylamin versetzt. Die Mischung wird 3 Stunden bei Raumtemperatur gerührt und über Nacht stehengelassen. Die Lösung wird eingeengt und mit Hexan/Essigester-Gemisch über Kieselgel chromatographiert.

Ausbeute: 1,36 g = 58 % der Theorie
Fp.: 220-222 °C

Elementaranalyse

| | |
|---|---|
| ber. (%): | C 34,84 H 3,19 N 22,16 S 8,46 Cl 18,70 |
| gef. (%): | C 34,80 H 3,64 N 21,99 S 8,13 Cl 18,33 |

b) 5-Amino-1,2,4-triazol-3-sulfonsäure-(N-methyl-2,6-dichloranilid)

9.80 g (22,8 mmol) 1-Acetyl-5-amino-1,2,4-triazol-3-sulfonsäure-2,6-dichloranilid, Pyridiniumsalz werden in 300 ml Dimethylformamid gelöst und 0,89 g (29,7 mmol) 80%iges Natriumhydrid portionsweise bei Raumtemperatur zugegeben. Man rührt 30 Minuten nach und fügt 1,7 ml (27,8 mmol) Methyljodid hinzu. Die Mischung wird über Nacht stehengelassen und eingeengt. Der Rückstand wird in 2 N Natriumhydroxid-Lösung aufgenommen und mit 3 N Salzsäure-Lösung schwach sauer gestellt. Die Kristalle werden abgesaugt, mit Wasser und Ether gewaschen und aus Acetonitril umkristallisiert.

Ausbeute: 3,4 g = 46 % der Theorie
Fp.: 254-255 °C

Elementaranalyse

| | |
|---|---|
| ber. (%): | C 33,55 H 2,82 N 21,74 S 9,95 Cl 22,01 |
| gef. (%): | C 33,35 H 3,10 N 21,63 S 10,08 Cl 22,18 |

**Beispiel 2**

5,6-Dimethyl-7-oxo-6,7-dihydro-[1,2,4]triazolo[1,5-a][1,3,5]triazin-2-sulfonsäure-2,6-dichloranilid

2.1 nach Verfahrensvariante E)

3,4 g (9,0 mmol) N-(2,6-Dichlorphenyl)-5-(1-ethoxyethyliden-amino)-1H-1,2,4-triazol-3-sulfonamid werden in 70 ml Tetrahydrofuran mit 0,66 ml (11,3 mmol) Methylisocyanat und 1,3 ml (9,3 mmol) Triethylamin 4 Stunden bei Raumtemperatur gerührt. Die Kristalle werden abgesaugt, mit Tetrahydrofuran/Wasser 4 : 1, Tetrahydrofuran und Ether gewaschen und im Vakuum getrocknet.

Ausbeute: 3,4 g = 97 % der Theorie
Fp.: 331-334 °C.

Elementaranalyse

| | |
|---|---|
| ber. (%): | C 37,03 H 2,59 N 21,59 S 8,24 Cl 18,22 |
| gef. (%): | C 37,21 H 2,60 N 21,22 S 8,54 Cl 18,35 |

2.2 nach Verfahrensvariante A)

0,88 g (5,4 mmol) 2,6-Dichloranilin werden in 11 ml Pyridin gelöst und mit 1,67 g (6,0 mmol) 5,6-Dimethyl-7-oxo-6,7-dihydro-[1,2,4]triazolo[1,5-a][1,3,5]triazin-2-sulfonsäurechlorid versetzt. Die Mischung wird 11 Stunden bei 50 ° C und 5 Stunden bei 75 ° C gerührt. Das Pyridin wird abdestilliert und der Rückstand über Kieselgel mit Hexan/Essigester chromatographiert.

Ausbeute: 0,23 g = 10 % der Theorie
Fp.: 331-334 ° C.

Herstellung der Ausgangsverbindungen zu Beispiel 2.1

a) N-(2,6-Dichlorphenyl)-5-(1-ethoxy-ethylidenamino)-1H-1,2,4-triazol-3-sulfonamid

6,16 g (20,0 mmol) 5-Amino-1H-1,2,4-triazol-3-sulfonsäure-2,6-dichloranilid werden in 50 ml Acetonitril mit 4,2 ml (23,0 mmol) Orthoessigsäuretriethylester und 3 Tropfen Eisessig 5 Stunden am Rückfluß erhitzt. Die Mischung wird abgekühlt und zur Trockne eingeengt. Der Rückstand wird aus Essigester umkristallisiert.

Ausbeute: 6,1 g = 80 % der Theorie
Fp.: 222-224 ° C

Elementaranalyse

ber. (%): C 38,10 H 3,46 N 18,52 S 8,48 Cl 18,75
gef. (%): C 38,09 H 3,63 N 18,64 S 8,57 Cl 18,89

Analog zu diesem Verfahren wurden auch die folgenden Verbindungen hergestellt:

| Name der Verbindung | Physikalische Konstante |
|---|---|
| N-(2,6-Dichlorphenyl)-5-(ethoxy-methylenamino)-1H-1,2,4-triazol-3-sulfonamid | Fp: 181-183°C |
| N-(2,6-Dichlorphenyl)-5-(1-ethoxy-propylidenamino)-1H-1,2,4-triazol-3-sulfonamid | Fp: 212-216°C |
| N-(2,6-Dichlorphenyl)-5-(ethoxy-benzylidenamino)-1H-1,2,4-triazol-3-sulfonamid | Fp: 213-217°C |
| N-(2,6-Dichlor-3-methylphenyl)-5-(1-ethoxy-ethylidenamino)-1H-1,2,4-triazol-3-sulfonamid | Fp: 202-204°C |
| N-(2-Methyl-6-nitrophenyl)-5-(1-ethoxy-ethylidenamino)-1H-1,2,4-triazol-3-sulfonamid | Fp: 195-197°C |
| N-(2-Chlor-6-fluorphenyl)-5-(1-ethoxy-ethylidenamino)-1H-1,2,4-triazol-3-sulfonamid | Fp: 214-215°C |
| N-(2,6-Difluorphenyl)-5-(1-ethoxy-ethylidenamino)-1H-1,2,4-triazol-3-sulfonamid | Fp: 187-188°C |
| N-(2,6-Dibromphenyl)-5-(1-ethoxy-ethylidenamino)-1H-1,2,4-triazol-3-sulfonamid | Fp: 184-187°C |

| Name der Verbindung | Physikalische Konstante |
|---|---|
| N-(2-Trifluormethylphenyl)-5-(1-ethoxy-ethylidenamino)-1H-1,2,4-triazol-3-sulfonamid | Fp: 124-125°C |
| N-Phenyl-5-(1-ethoxy-ethylidenamino)-1H-1,2,4-triazol-3-sulfonamid | Fp: 164-166°C |
| N-(2-Chlorphenyl)-5-(1-ethoxy-ethylidenamino)-1H-1,2,4-triazol-3-sulfonamid | Fp: 150-153°C |
| N-(2-Methoxyphenyl)-5-(1-ethoxy-ethylidenamino)-1H-1,2,4-triazol-3-sulfonamid | Fp: 138-140°C |

b) 5-Amino-1H-1,2,4-triazol-3-sulfonsaure-2,6-dichloranilid

53.6 g (0.12 mol) 1-Acetyl-5-amino-1,2,4-triazol-3-sulfonsäure-2,6-dichloranilid, Pyridiniumsalz werden in 200 ml 2 n NaOH gelöst und 10 Minuten bei Raumtemperatur gerührt. Unter Eiskühlung wird mit 2 n Salzsäure-Lösung ein pH-Wert 5 eingestellt. Die Kristalle werden abgesaugt, mit wenig Wasser und Ether gewaschen und im Vakuum getrocknet.

Ausbeute: 35.4 g = 92 % der Theorie
Fp.: 265-267 ° C

Elementaranalyse

ber. (%): C 31,18 H 2,29 N 22,73 S 10,41 Cl 23,01
gef. (%): C 31,38 H 2,40 N 22,37 S 10,23 Cl 22,23

c) 1-Acetyl-5-amino-1,2,4-triazol-3-sulfonsaure-2,6-dichloranilid, Pyridiniumsalz

42.63 g (0,26 mol) 2,6-Dichloranilin werden in 350 ml Pyridin mit 54 g (0,24 mol) 1-Acetyl-5-amino-1,2,4-trizolsulfonsäurechlorid versetzt und 12 Stunden bei 60 ° C gerührt. Nach Abkuhlen werden die Kristalle abgesaugt, mit wenig Pyridin und Ether gewaschen und im Vakuum getrocknet.

Ausbeute: 53,6 g = 52 % der Theorie
Fp.: 213-215 ° C

Elementaranalyse

ber. (%): C 41,96 H 3,29 N 19,58 S 7,47 Cl 16,52
gef. (%): C 41,89 H 3,33 N 19,36 S 7,46 Cl 15,97

d) 1-Acetyl-5-amino-1,2,4-triazol-3-sulfonsäurechlorid

112 g (0,45 mol) 1-Acetyl-5-amino-3-benzylthio-1,2,4-triazol werden in 1 Liter Wasser/Eisessig (1:1-Gemisch) suspendiert. Bei -10 ° C wird 2 Stunden Chlor eingeleitet, so daß die Innentemperatur unter 0 ° C bleibt. Die entstandenen Kristalle werden abgesaugt, mit wenig Wasser und Pentan gewaschen und getrocknet.

Ausbeute: 74,5 g = 73,5 % der Theorie
Fp.: 166-168 ° C.

e) 1-Acetyl-5-amino-3-benzylthio-1,2,4-triazol

Zu 30 g (0,145 mol) 5-Amino-3-benzylthio-1H-1,2,4-triazol und 25 ml Triethylamin in 300 ml Methylenchlorid werden bei 0 °C 12,57 g (0,16 mol) Acetylchlorid in 30 ml Methylenchlorid getropft. Bei Raumtemperatur wird 30 Minuten nachgerührt, anschließend mit 160 ml 1 n Natronlauge versetzt und das Produkt mit Methylenchlorid extrahiert. Nach dem Trocken wird aus Essigester umkristallisiert.

| | |
|---|---|
| Ausbeute: | 33 g = 91 % der Theorie |
| Fp.: | 146 °C |

Elementaranalyse

| | |
|---|---|
| ber. (%): | C 53,20 H 4,87 N 22,56 S 12,91 |
| gef. (%): | C 53.51 H 5.36 N 22,81 S 12,86 |

f) 5-Amino-3-benzylthio-1H-1,2,4-triazol

40,36 g (0,35 mol) 3-Amino-5-mercapto-1,2,4-triazol werden in 450 ml Ethanol mit 13,9 g (0,35 mol) festem Natriumhydroxid bei Raumtemperatur versetzt. Bei Raumtemperatur wird 44,30 g (0,35 mol) Benzylchlorid zugetropft und die Lösung über Nacht nachgerührt. Es wird vom Salz abfiltriert, Ethanol abdestilliert und der Rückstand aus Essigester umkristallisiert und getrocknet.

| | |
|---|---|
| Ausbeute: | 64,97 g = 90 % der Theorie |
| Fp.: | 104 °C |

Elementaranalyse

| | |
|---|---|
| ber. (%): | C 52,40 H 4,88 N 27,16 S 15,54 |
| gef. (%): | C 52,10 H 4,84 N 27,48 S 15,11 |

<u>Herstellung der Ausgangsverbindungen zu Beispiel 2.2</u>

a) 5,6-Dimethyl-7-oxo-6,7-dihydro[1,2,4]triazolo[1,5-a][1,3,5]triazin-2-sulfonsäurechlorid

3,00 g (10,4 mmol) 2-Benzylthio-5,6-dimethyl-6,7-dihydro-[1,2,4]triazolo-[1,5-a][1,3,5]triazin-7-on werden in 50 ml Wasser/Essigsäure (1:1-Gemisch) suspendiert. Bei 5 bis 15 °C wird 45 Minuten Chlor eingeleitet und anschließend 1 Stunde gerührt. Die entstandenen Kristalle werden abgesaugt, mit wenig Wasser und Ether gewaschen und bei 50 °C im Vakuum getrocknet.

| | |
|---|---|
| Ausbeute: | 2,22 g = 80.7 % der Theorie |
| Fp.: | 206-207 °C. |

Elementaranalyse

| | |
|---|---|
| ber. (%): | C 27,33 H 2,29 N 26,56 S 12,16 Cl 13,45 |
| gef. (%): | C 27,51 H 2,69 N 26,33 S 12,13 Cl 13,30 |

b) 2-Benzylthio-5,6-dimethyl-6,7-dihydro-[1,2,4]triazolo[1,5-a][1,3,5]triazin-7-on

8,50 g (30,8 mmol) 3-Benzylthio-5-(1-ethoxy-ethylidenamino)-1H-1,2,4-triazol werden in 250 ml Tetrahydrofuran gelöst und mit 2,23 g (38,5 mmol) Methylisocyanat und 3,73 g (36,9 mmol) Triethylamin versetzt. Man rührt 5 Stunden bei 70 °C, destilliert das Lösungsmittel ab und versetzt den Rückstand mit Ether. Die Kristalle werden abgesaugt und aus Toluol umkristallisiert.

| | |
|---|---|
| Ausbeute: | 4,40 g = 49,8 % der Theorie |
| Fp.: | 127-128 °C |

Elementaranalyse

| | |
|---|---|
| ber. (%): | C 54,34 H 4,56 N 24,27 S 11,16 |
| gef. (%): | C 54,30 H 4,79 N 24,33 S 10,96 |

c) 3-Benzylthio-5-(1-ethoxy-ethylidenamino)-1H-1,2,4-triazol

10,00 g (48,5 mmol) 5-Amino-3-benzylthio-1H-1,2,4-triazol werden in 120 ml Acetonitril suspendiert und mit 8,83 g (53,3 mmol) Orthoessigsäuretriethylester und 3 Tropfen Eisessig versetzt. Man erhitzt 6,5 Stunden am Rückfluß, engt zur Trockene ein, nimmt in der Hitze in Hexan auf, filtriert und engt wieder zur Trockne ein.

| | |
|---|---|
| Ausbeute: | 10,89 g = 81,3 % der Theorie |
| Fp.: | 89-90 ° C |

Elementaranalyse

| | |
|---|---|
| ber. (%): | C 56,50 H 5,84 N 20,27 S 11,60 |
| gef. (%): | C 56,59 H 5,37 N 20,58 S 11,92 |

**Beispiel 3**

5,6-Dimethyl-7-thioxo-6,7-dihydro-[1,2,4]triazolo[1,5-a][1,3,5]triazin-2-sulfonsäure-2,6-dichloranilid, Triethylammonium-Salz

(nach Verfahrensvariante E))

7.56 g (0,02 mol) N-(2,6-Dichlorphenyl)-5-(1-ethoxyethyliden-amino)-1H-1,2,4-triazol-3-sulfonamid werden in 70 ml Tetrahydrofuran mit 1,7 ml (0,025 mol) Methylisothiocyanat und 3,5 ml (0,025 mol) Triethylamin versetzt und 8 Stunden bei 50 ° C gerührt. Die Kristalle werden abgesaugt, mit Tetrahydrofuran und Ether gewaschen und im Vakuum getrocknet.

| | |
|---|---|
| Ausbeute: | 5,4 g = 53 % der Theorie |
| Fp.: | 217-218 ° C. |

Elementaranalyse

| | |
|---|---|
| ber. (%): | C 42,68 H 4,97 N 19,36 S 12,66 Cl 14,00 |
| gef. (%): | C 42,83 H 4,82 N 19,34 S 12,69 Cl 14,13 |

**Beispiel 4**

5,6-Dimethyl-7-thioxo-6,7-dihydro-[1,2,4]triazolo[1,5-a][1,3,5]triazin-2-sulfonsäure-2,6-dichloranilid

1,7 g (3,4 mmol) 5,6-Dimethyl-7-thioxo-6,7-dihydro-[1,2,4]triazolo[1,5-a]-[1,3,5]triazin-2-sulfonsäure-2,6-dichloranilid, Triethylammonium-Salz werden in 100 ml Wasser suspendiert und mit 1 N Salzsäure-Lösung sauer gestellt. Die Kristalle werden abgesaugt, mit Wasser und Ether gewaschen und im Vakuum getrocknet.

| | |
|---|---|
| Ausbeute: | 1,3 g = 94 % der Theorie |
| Fp.: | 301-302 ° C. |

Elementaranalyse

| | |
|---|---|
| ber. (%): | C 35,56 H 2,49 N 20,74 S 15,82 Cl 17.50 |
| gef. (%): | C 35,18 H 2,70 N 20,28 S 16,19 Cl 17,74 |

Analog zu den Beispielen 1 bis 4 lassen sich auch die folgenden erfindungsgemaßen Verbindungen herstellen:

| Beispiel | Name der Verbindung | Physikalische Konstante |
|---|---|---|
| 5 | 5,6-Dimethyl-7-thioxo-6,7-dihydro-[1,2,4]triazolo[1,5-a][1,3,5]triazin-2-sulfonsäure-2,6-dichlor-3-methyl-anilid | Fp.: 241-242°C |
| 6 | 5,6-Dimethyl-7-thioxo-6,7-dihydro-[1,2,4]triazolo[1,5-a][1,3,5]triazin-2-sulfonsaure-2-methyl-6-nitroanilid | Fp.: 242-244°C |
| 7 | 5,6-Dimethyl-7-thioxo-6,7-dihydro-[1,2,4]triazolo[1,5-a][1,3,5]triazin-2-sulfonsäure-2-chlor-6-fluoranilid | Fp.: 274-275°C |
| 8 | 5,6-Dimethyl-7-thioxo-6,7-dihydro-[1,2,4]triazolo[1,5-a][1,3,5]triazin-2-sulfonsäure-2,6-difluoranilid | Fp.: 284-285°C |
| 9 | 5,6-Dimethyl-7-thioxo-6,7-dihydro-[1,2,4]triazolo[1,5-a][1,3,5]triazin-2-sulfonsäure-2,6-dibromanilid | Fp.: 312-314°C |
| 10 | 5,6-Dimethyl-7-thioxo-6,7-dihydro-[1,2,4]triazolo[1,5-a][1,3,5]triazin-2-sulfonsäure-2-trifluormethylanilid | Fp.: 233-235°C |

| Beispiel | Name der Verbindung | Physikalische Konstante |
| --- | --- | --- |
| 11 | 5,6-Dimethyl-7-thioxo-6,7-dihydro-[1,2,4]triazolo[1,5-a][1,3,5]triazin-2-sulfonsäure-anilid | Fp.: 231-233°C |
| 12 | 5,6-Dimethyl-7-thioxo-6,7-dihydro-[1,2,4]triazolo[1,5-a][1,3,5]triazin-2-sulfonsäure-2-chloranilid | Fp.: 200-206°C |
| 13 | 5,6-Dimethyl-7-thioxo-6,7-dihydro-[1,2,4]triazolo[1,5-a][1,3,5]triazin-2-sulfonsäure-2-methoxy-anilid | Fp.: 198-200°C |
| 14 | 5,6-Dimethyl-7-oxo-6,7-dihydro-[1,2,4]triazolo-[1,5-a][1,3,5]triazin-2-sulfonsäure-2,6-dichlor-3-methylanilid | Fp.: 336-338°C |
| 15 | 5,6-Dimethyl-7-oxo-6,7-dihydro-[1,2,4]triazolo-[1,5-a][1,3,5]triazin-2-sulfonsäure-2-methyl-6-nitroanilid | |
| 16 | 5,6-Dimethyl-7-oxo-6,7-dihydro-[1,2,4]triazolo-[1,5-a][1,3,5]triazin-2-sulfonsäure-2-chlor-6-fluoranilid | Fp.: 304-306°C |
| 17 | 5,6-Dimethyl-7-oxo-6,7-dihydro-[1,2,4]triazolo-[1,5-a][1,3,5]triazin-2-sulfonsäure-2,6-difluoranilid | Fp.: 298-300°C |
| 18 | 5,6-Dimethyl-7-oxo-6,7-dihydro-[1,2,4]triazolo-[1,5-a][1,3,5]triazin-2-sulfonsäure-2,6-dibromanilid | Fp.: 327-332°C |
| 19 | 5,6-Dimethyl-7-oxo-6,7-dihydro-[1,2,4]triazolo-[1,5-a][1,3,5]triazin-2-sulfonsäure-2-trifluormethylanilid | Fp.: 268-270°C |

| Beispiel | Name der Verbindung | Physikalische Konstante |
|---|---|---|
| 20 | 5,6-Dimethyl-7-oxo-6,7-dihydro-[1,2,4]triazolo-[1,5-a][1,3,5]triazin-2-sulfonsäure-anilid | |
| 21 | 5,6-Dimethyl-7-oxo-6,7-dihydro-[1,2,4]triazolo-[1,5-a][1,3,5]triazin-2-sulfonsaure-2-chlor-anilid | Fp.: 202-206 °C |
| 22 | 5,6-Dimethyl-7-oxo-6,7-dihydro-[1,2,4]triazolo-[1,5-a][1,3,5]triazin-2-sulfonsäure-2-methoxy-anilid | Fp.: 358-359 °C |
| 23 | 6-Ethyl-5-methyl-7-thioxo-6,7-dihydro-[1,2,4]-triazolo[1,5-a][1,3,5]triazin-2-sulfonsäure-2,6-dichloranilid, Triethylammonium-Salz | Fp.: 179-180 °C |
| 24 | 6-Ethyl-5-methyl-7-oxo-6,7-dihydro-[1,2,4]-triazolo[1,5-a][1,3,5]triazin-2-sulfonsäure-2,6-dichloranilid, Triethylammonium-Salz | Fp.: 185-186 °C |
| 25 | 5-Methyl-6-phenyl-7-thioxo-6,7-dihydro-[1,2,4]-triazolo[1,5-a][1,3,5]triazin-2-sulfonsäure-2,6-dichloranilid, Triethylammonium-Salz | Fp.: 233-235 °C |
| 26 | 5-Methyl-6-phenyl-7-oxo-6,7-dihydro-[1,2,4]-triazolo[1,5-a][1,3,5]triazin-2-sulfonsäure-2,6-dichloranilid | Fp.: 298-300 °C |
| 27 | 6-Methyl-7-thioxo-6,7-dihydro-[1,2,4]triazolo-[1,5-a][1,3,5]triazin-2-sulfonsäure-2,6-dichlor-anilid | |
| 28 | 6-Methyl-7-oxo-6,7-dihydro-[1,2,4]triazolo-[1,5-a][1,3,5]triazin-2-sulfonsäure-2,6-dichlor-anilid | Fp.: 296-300 °C |

| Beispiel | Name der Verbindung | Physikalische Konstante |
|---|---|---|
| 29 | 6-Ethyl-7-thioxo-6,7-dihydro-[1,2,4]triazolo-[1,5-a][1,3,5]triazin-2-sulfonsäure-2,6-dichloranilid | |
| 30 | 6-Ethyl-7-oxo-6,7-dihydro-[1,2,4]triazolo-[1,5-a][1,3,5]triazin-2-sulfonsäure-2,6-dichloranilid | Fp.: 285-287°C |
| 31 | 6-Phenyl-7-thioxo-6,7-dihydro-[1,2,4]triazolo[1,5-a][1,3,5]triazin-2-sulfonsäure-2,6-dichloranilid | |
| 32 | 6-Phenyl-7-oxo-6,7-dihydro-[1,2,4]triazolo-[1,5-a][1,3,5]triazin-2-sulfonsäure-2,6-dichloranilid | Fp.: 262-265°C |
| 33 | 5-Ethyl-6-methyl-7-thioxo-6,7-dihydro-[1,2,4]-triazolo[1,5-a][1,3,5]triazin-2-sulfonsaure-2,6-dichloranilid, Triethylammonium-Salz | Fp.: 260-262°C |
| 34 | 5-Ethyl-6-methyl-7-oxo-6,7-dihydro-[1,2,4]-triazolo[1,5-a][1,3,5]triazin-2-sulfonsäure-2,6-dichloranilid, Triethylammonium-Salz | Fp.: 289-291°C |
| 35 | 5,6-Diethyl-7-thioxo-6,7-dihydro-[1,2,4]-triazolo[1,5-a][1,3,5]triazin-2-sulfonsäure-2,6-dichloranilid, Triethylammonium-Salz | Fp.: 185-187°C |
| 36 | 5,6-Diethyl-7-oxo-6,7-dihydro-[1,2,4]triazolo[1,5-a][1,3,5]triazin-2-sulfonsäure-2,6-dichloranilid, Triethylammonium-Salz | Fp.: 277-280°C |

| Beispiel | Name der Verbindung | Physikalische Konstante |
|---|---|---|
| 37 | 5-Ethyl-6-phenyl-7-thioxo-6,7-dihydro-[1,2,4]-triazolo[1,5-a][1,3,5]triazin-2-sulfonsäure-2,6-dichloranilid, Triethylammonium-Salz | Fp.: 219-221°C |
| 38 | 5-Ethyl-6-phenyl-7-oxo-6,7-dihydro-[1,2,4]-triazolo[1,5-a][1,3,5]triazin-2-sulfonsäure-2,6-dichloranilid, Triethylammonium-Salz | Fp.: 291-293°C |
| 39 | 6-Methyl-5-phenyl-7-thioxo-6,7-dihydro-[1,2,4]triazolo[1,5-a][1,3,5]triazin-2-sulfonsäure-2,6-dichloranilid, Hydrat | Fp.: 270-272°C |
| 40 | 6-Methyl-5-phenyl-7-oxo-6,7-dihydro-[1,2,4]triazolo[1,5-a][1,3,5]triazin-2-sulfonsäure-2,6-dichloranilid, Triethyl-ammonium-Salz | Fp.: 201-203°C |
| 41 | 6-Ethyl-5-phenyl-7-thioxo-6,7-dihydro-[1,2,4]triazolo[1,5-a][1,3,5]triazin-2-sulfonsäure-2,6-dichloranilid | |
| 42 | 6-Ethyl-5-phenyl-7-oxo-6,7-dihydro-[1,2,4]triazolo[1,5-a][1,3,5]triazin-2-sulfonsäure-2,6-dichloranilid, Triethyl-ammonium-Salz | Fp.: 209-212°C |
| 43 | 5,6-Diphenyl-7-thioxo-6,7-dihydro-[1,2,4]-triazolo[1,5-a][1,3,5]triazin-2-sulfon-säure-2,6-dichloranilid | |
| 44 | 5,6-Diphenyl-7-oxo-6,7-dihydro-[1,2,4]-triazolo[1,5-a][1,3,5]triazin-2-sulfon-säure-2,6-dichloranilid, Triethylammo-nium-Salz | Fp.: 225-227°C |

| Beispiel | Name der Verbindung | Physikalische Konstante |
|---|---|---|
| 45 | 5,6-Dimethyl-7-oxo-6,7-dihydro-[1,2,4]-triazolo[1,5-a][1,3,5]triazin-2-sulfonsäure-(N-ethyl-2,6-dichloranilid) | Fp.: 286-288°C |
| 46 | 5,6-Dimethyl-7-thioxo-6,7-dihydro-[1,2,4]-triazolo[1,5-a][1,3,5]triazin-2-sulfonsäure-(N-methyl-2,6-dichloranilid) | Fp.: 296-298°C |
| 47 | 5,6-Dimethyl-7-oxo-6,7-dihydro-[1,2,4]triazolo[1,5-a][1,3,5]triazin-2-sulfonsäure-2,6-dichloranilid, Triethylammonium-Salz | Fp.: 298-300°C |
| 48 | 5,6-Dimethyl-7-thioxo-6,7-dihydro-[1,2,4]-triazolo[1,5-a][1,3,5]triazin-2-sulfonsäure-2,6-dichloranilid, Natrium-Salz | |
| 49 | 5,6-Dimethyl-7-thioxo-6,7-dihydro-[1,2,4]triazolo[1,5-a][1,3,5]triazin-2-sulfonsäure-2,6-dichlor-3-methyl-anilid, Triethylammonium-Salz | Fp.: 208-210°C |
| 50 | 5,6-Dimethyl-7-thioxo-6,7-dihydro-[1,2,4]triazolo[1,5-a][1,3,5]triazin-2-sulfonsäure-2-chlor-6-fluoranilid, Triethylammonium-Salz | Fp.: 173-175°C |
| 51 | 5,6-Dimethyl-7-thioxo-6,7-dihydro-[1,2,4]triazolo[1,5-a][1,3,5]triazin-2-sulfonsäure-2,6-difluoranilid, Triethylammonium-Salz | Fp.: 188-190°C |
| 52 | 5,6-Dimethyl-7-thioxo-6,7-dihydro-[1,2,4]triazolo[1,5-a][1,3,5]triazin-2-sulfonsäure-2,6-dibromanilid, Triethylammonium-Salz | Fp.: 301-305°C |

| Beispiel | Name der Verbindung | Physikalische Konstante |
|---|---|---|
| 53 | 5,6-Dimethyl-7-thioxo-6,7-dihydro-[1,2,4]triazolo[1,5-a][1,3,5]triazin-2-sulfonsäure-2-methyl-6-nitroanilid, Triethylammonium-Salz | Fp.: 193-195°C |
| 54 | 5,6 Dimethyl-7-thioxo-6,7-dihydro-[1,2,4]triazolo[1,5-a][1,3,5]triazin-2-sulfonsäure-2-trifluormethylanilid, Triethylammonium-Salz | Fp.: 209-211°C |
| 55 | 5,6-Dimethyl-7-oxo-6,7-dihydro-[1,2,4]triazolo[1,5-a][1,3,5]triazin-2-sulfonsäure-2,6-dichlor-3-methylanilid, Triethylammonium-Salz | Fp.: 306-309°C |
| 56 | 5,6-Dimethyl-7-oxo-6,7-dihydro-[1,2,4]triazolo[1,5-a][1,3,5]triazin-2-sulfonsäure-2-chlor-6-fluoranilid, Triethylammonium-Salz | Fp.: 301-303°C |
| 57 | 5,6-Dimethyl-7-oxo-6,7-dihydro-[1,2,4]triazolo[1,5-a][1,3,5]triazin-2-sulfonsäure-2,6-difluoranilid, Triethylammonium-Salz | Fp.: 300-302°C |
| 58 | 5,6-Dimethyl-7-oxo-6,7-dihydro-[1,2,4]triazolo[1,5-a][1,3,5]triazin-2-sulfonsäure-2-jodanilid | Fp.: 213-218°C |
| 59 | 5,6-Dimethyl-7-oxo-6,7-dihydro-[1,2,4]triazolo[1,5-a][1,3,5]triazin-2-sulfonsäure-2-chlor-6-methylanilid, Triethylammonium-Salz | Fp.: 250-252°C |

| Beispiel | Name der Verbindung | Physikalische Konstante |
| --- | --- | --- |
| 60 | 5,6-Dimethyl-7-thioxo-6,7-dihydro-[1,2,4]triazolo[1,5-a][1,3,5]triazin-2-sulfonsäure-2-bromanilid | Fp.: 188-190°C |
| 61 | 5,6-Dimethyl-7-thioxo-6,7-dihydro-[1,2,4]triazolo[1,5-a][1,3,5]triazin-2-sulfonsäure-2-chlor-6-methylanilid | Fp.: 266-267°C |
| 62 | 5,6-Dimethyl-7-thioxo-6,7-dihydro-[1,2,4]triazolo[1,5-a][1,3,5]triazin-2-sulfonsäure-2-chlor-6-methylanilid, Triethylammonium-Salz | Fp.: 240-242°C |
| 63 | 5,6-Dimethyl-7-thioxo-6,7-dihydro-[1,2,4]triazolo[1,5-a][1,3,5]triazin-2-sulfonsäure-2-chlor-6-ethylanilid | Fp.: 246-248°C |
| 64 | 5,6-Dimethyl-7-thioxo-6,7-dihydro-[1,2,4]triazolo[1,5-a][1,3,5]triazin-2-sulfonsäure-2-ethoxycarbonylanilid | Fp.: 163-165°C |
| 65 | 5,6-Dimethyl-7-thioxo-6,7-dihydro-[1,2,4]triazolo[1,5-a][1,3,5]triazin-2-sulfonsäure-2-methylthioanilid, Hydrat | Fp.: 140-141°C |
| 66 | 5,6-Dimethyl-7-thioxo-6,7-dihydro-[1,2,4]triazolo[1,5-a][1,3,5]triazin-2-sulfonsäure-2-ethoxycarbonyl-6-methylanilid | Fp.: 168-170°C |
| 67 | 5,6-Dimethyl-7-thioxo-6,7-dihydro-[1,2,4]triazolo[1,5-a][1,3,5]triazin-2-sulfonsäure-2,3-dimethyl-6-nitroanilid, Hydrat | Fp.: 281-283°C |

| Beispiel | Name der Verbindung | Physikalische Konstante |
|---|---|---|
| 68 | 5,6-Dimethyl-7-thioxo-6,7-dihydro-[1,2,4]triazolo[1,5-a][1,3,5]triazin-2-sulfonsäure-2-methoxycarbonyl-6-methylanilid | Fp.: 212-213°C |
| 69 | 5,6-Dimethyl-7-oxo-6,7-dihydro-[1,2,4]triazolo[1,5-a][1,3,5]triazin-2-sulfonsäure-2-bromanilid | Fp.: 183-187°C |
| 70 | 5,6-Dimethyl-7-oxo-6,7-dihydro-[1,2,4]triazolo[1,5-a][1,3,5]triazin-2-sulfonsäure-2-methoxycarbonylanilid | Fp.: 244-246°C |
| 71 | 5,6-Dimethyl-7-oxo-6,7-dihydro-[1,2,4]triazolo[1,5-a][1,3,5]triazin-2-sulfonsäure-2-ethoxycarbonylanilid | Fp.: 233-236°C |
| 72 | 5,6-Dimethyl-7-oxo-6,7-dihydro-[1,2,4]triazolo[1,5-a][1,3,5]triazin-2-sulfonsäure-2-isopropoxycarbonylanilid | Fp.: 244-246°C |
| 73 | 5,6-Dimethyl-7-oxo-6,7-dihydro-[1,2,4]triazolo[1,5-a][1,3,5]triazin-2-sulfonsäure-2-chlor-6-ethylanilid | Fp.: 275-277°C |
| 74 | 5,6-Dimethyl-7-oxo-6,7-dihydro-[1,2,4]triazolo[1,5-a][1,3,5]triazin-2-sulfonsäure-2-acetylanilid | Fp.: 225-227°C |
| 75 | 5,6-Dimethyl-7-oxo-6,7-dihydro-[1,2,4]triazolo[1,5-a][1,3,5]triazin-2-sulfonsäure-2-isopropyl-6-methylanilid | Fp.: 284-285°C |

| Beispiel | Name der Verbindung | Physikalische Konstante |
|---|---|---|
| 76 | 5,6-Dimethyl-7-oxo-6,7-dihydro-[1,2,4]triazolo[1,5-a][1,3,5]triazin-2-sulfonsäure-2-methoxycarbonyl-6-methylanilid | Fp.: 257-258°C |
| 77 | 5,6-Dimethyl-7-oxo-6,7-dihydro-[1,2,4]triazolo[1,5-a][1,3,5]triazin-2-sulfonsäure-N-methylaminocarbonyl-2-isopropyl-6-methylanilid | Fp.: 253-254°C |
| 78 | 5,6-Dimethyl-7-oxo-6,7-dihydro-[1,2,4]triazolo[1,5-a][1,3,5]triazin-2-sulfonsäure-2-fluoranilid | Fp.: 229-231°C |
| 79 | 5,6-Dimethyl-7-oxo-6,7-dihydro-[1,2,4]triazolo[1,5-a][1,3,5]triazin-2-sulfonsäure-2-brom-4-fluoranilid, Hydrat | Fp.: 234-235°C |
| 80 | 5-Methyl-7-oxo-6,7-dihydro-[1,2,4]triazolo-[1,5-a][1,3,5]triazin-2-sulfonsäure-2,6-dichloranilid | Fp.: 298-300°C |
| 81 | 5,6-Dimethyl-7-thioxo-6,7-dihydro-[1,2,4]triazolo[1,5-a][1,3,5]triazin-2-sulfonsäure-2-jodanilid | Fp.: 192-194°C |
| 82 | 5,6-Dimethyl-7-thioxo-6,7-dihydro-[1,2,4]triazolo[1,5-a][1,3,5]triazin-2-sulfonsäure-2-isopropyl-6-methylanilid | Fp.: 269-271°C |
| 83 | 5,6-Dimethyl-7-thioxo-6,7-dihydro-[1,2,4]triazolo[1,5-a][1,3,5]triazin-2-sulfonsäure-2-brom-6-chlor-4-fluoranilid | Fp.: 271-273°C |

| Beispiel | Name der Verbindung | Physikalische Konstante |
|---|---|---|
| 84 | 5,6-Dimethyl-7-thioxo-6,7-dihydro-[1,2,4]triazolo[1,5-a][1,3,5]triazin-2-sulfonsäure-2-brom-6-methoxyanilid | Fp.: 229-231°C |
| 85 | 5,6-Dimethyl-7-oxo-6,7-dihydro-[1,2,4]triazolo[1,5-a][1,3,5]triazin-2-sulfonsäure-2,6-dimethylanilid, Hydrat | Fp.: 263-265°C |
| 86 | 5,6-Dimethyl-7-thioxo-6,7-dihydro-[1,2,4]triazolo[1,5-a][1,3,5]triazin-2-sulfonsäure-4-brom-2,6-dichloranilid, Hydrat | Fp.: 283-285°C |
| 87 | 5,6-Dimethyl-7-thioxo-6,7-dihydro-[1,2,4]triazolo[1,5-a][1,3,5]triazin-2-sulfonsäure-2-methoxycarbonyl-3,6-dimethylanilid | Fp.: 222-224°C |
| 88 | 5,6-Dimethyl-7-thioxo-6,7-dihydro-[1,2,4]triazolo[1,5-a][1,3,5]triazin-2-sulfonsäure-2-methoxycarbonyl-5,6-dimethylanilid | Fp.: 194-195°C |
| 89 | 5,6-Dimethyl-7-thioxo-6,7-dihydro-[1,2,4]triazolo[1,5-a][1,3,5]triazin-2-sulfonsäure-2-brom-6-chloranilid | Fp.: 304-305°C |
| 90 | 5,6-Dimethyl-7-oxo-6,7-dihydro-[1,2,4]triazolo[1,5-a][1,3,5]triazin-2-sulfonsäure-2-methoxycarbonyl-3,6-dimethylanilid | Fp.: 225-227°C |
| 91 | 5,6-Dimethyl-7-oxo-6,7-dihydro-[1,2,4]triazolo[1,5-a][1,3,5]triazin-2-sulfonsäure-2-methoxycarbonyl-5,6-dimethylanilid | Fp.: 216-218°C |

| Beispiel | Name der Verbindung | Physikalische Konstante |
|---|---|---|
| 92 | 5,6-Dimethyl-7-oxo-6,7-dihydro-[1,2,4]triazolo[1,5-a][1,3,5]triazin-2-sulfonsäure-2-brom-6-chloranilid | Fp.: 321-322 °C |
| 93 | 5,6-Dimethyl-7-oxo-6,7-dihydro-[1,2,4]triazolo[1,5-a][1,3,5]triazin-2-sulfonsäure-2-ethoxycarbonyl-6-methylanilid | Fp.: 195-197 °C |

Die nachfolgenden Beispiele erläutern die Anwendungsmöglichkeiten der erfindungsgemäßen Verbindungen:

Beispiel A

Im Gewächshaus wurden die in der Tabelle aufgeführten Verbindungen in einer Aufwandmenge von 1,0 kg Wirkstoff/ha, suspendiert in 500 Liter Wasser/ha, auf Testpflanzenarten der Gattungen Helianthus und Chrysanthemum im Vor- und Nachauflaufverfahren gespritzt.

Die Schädigung der Unkräuter wurde in einem Boniturschema von 0 bis 4 drei Wochen nach der Behandlung bonitiert, wobei bedeutet:

0 = keine Wirkung
1 = mittlere Wuchshemmung
2 = starke Wuchshemmung
3 = völlige Wuchshemmung
4 = total vernichtet

Es ergab sich, daß die erfindungsgemäßen Verbindungen der Beispiele 1 bis 14, 16 bis 19, 21 bis 26, 28, 30, 32 bis 40, 42, 44 bis 47 und 49 bis 93 in diesem Test eine 100%ige Vernichtung (= 4) sowohl im Vorauflauf als auch im Nachauflauf bei den Testpflanzenarten bewirkten.

Beispiel B

Samen mono- und dikotyler Unkräuter sowie die Kulturpflanzen Weizen (Triticum aestivum), Gerste (Hordeum distichum) und Reis (Oryza sativa) wurden in Töpfe mit humushaltigem Sandboden ausgelegt und mit Erde abgedeckt. Die aufgeführten erfindungsgemäßen Verbindungen wurden als Suspension mit 500 Liter Wasser/ha in einer Aufwandmenge von 0,1 kg Wirkstoff/ha vor dem Auflaufen der Pflanzen auf die Erdoberfläche appliziert.

Nach der Behandlung wurden die Versuchstöpfe im Gewächshaus aufgestellt und die Versuchspflanzen unter guten Wuchsbedingungen kultiviert. View Wochen nach der Behandlung wurden die Pflanzenschäden bonitiert. Als Vergleich dienten dabei unbehandelte Kontrollen.

Wie aus der Tabelle ersichtlich wird, wurden alle Unkrauter vernichtet ohne Schädigung der Kulturpflanzen.

In der folgenden Tabelle bedeuten:

0 = keine Wirkung
4 = Vernichtung der Pflanzen
Tr = Triticum aestivum
Ho = Hordeum distichum
Or = Oryza sativa
Se = Setaria sp.
He = Helianthus sp.
St = Stellaria sp.
Ab = Abutilon sp.

Vi = Viola sp.
Br = Brassica sp.
So = Solanum sp.
Ma = Matricaria sp.
Cy = Cyperus sp.
Ec = Echinochloa sp.

| Erfindungsgemäße Verbindung | Tr | Ho | Or | Se | He | St | Ab | Vi | Br | So | Ma | Cy | Ec |
|---|---|---|---|---|---|---|---|---|---|---|---|---|---|
| Beispiel 3 | 0 | 0 | 0 | 4 | 4 | 4 | 4 | 4 | 4 | 4 | 4 | 3 | 4 |
| Beispiel 4 | 0 | 0 | 0 | 4 | 4 | 4 | 4 | 4 | 4 | 4 | 4 | 3 | 4 |
| Kontrolle | 0 | 0 | 0 | 0 | 0 | 0 | 0 | 0 | 0 | 0 | 0 | 0 | 0 |

Beispiel C

Samen mono- und dikotyler Unkräuter sowie die Kulturpflanzen Weizen (Triticum aestivum), Gerste (Hordeum distichum) und Reis (Oryza sativa) wurden in Töpfe mit humushaltigem Sandboden ausgelegt und mit Erde abgedeckt. Die aufgeführten erfindungsgemäßen Verbindungen wurden als Suspension mit 500 Liter Wasser/ha in einer Aufwandmenge von 0,1 kg Wirkstoff/ha nach dem Auflaufen der Pflanzen auf die Erdoberfläche appliziert.

Nach der Behandlung wurden die Versuchstöpfe im Gewächshaus aufgestellt und die Versuchspflanzen unter guten Wuchsbedingungen kultiviert. Zwei Wochen nach der Behandlung wurden die Pflanzenschaden bonitiert. Als Vergleich dienten dabei unbehandelte Kontrollen.

Wie aus der Tabelle ersichtlich wird, wurden alle Unkräuter vernichtet ohne Schädigung der Kulturpflanzen.

In der folgenden Tabelle bedeuten:

0 = keine Wirkung
4 = Vernichtung der Pflanzen
Tr = Triticum aestivum
Ho = Hordeum distichum
Or = Oryza sativa
Se = Setaria sp.
He = Helianthus sp.
St = Stellaria sp.
Ab = Abutilon sp.
Vi = Viola sp.
Br = Brassica sp.
So = Solanum sp.
Ma = Matricaria sp.
Cy = Cyperus sp.
Ec = Echinochloa sp.

| Erfindungsgemäße Verbindung | Tr | Ho | Or | Se | He | St | Ab | Vi | Br | So | Ma | Cy | Ec |
|---|---|---|---|---|---|---|---|---|---|---|---|---|---|
| Beispiel 3 | 0 | 0 | 0 | 4 | 4 | 4 | 4 | 4 | 4 | 4 | 4 | 3 | 4 |
| Kontrolle | 0 | 0 | 0 | 0 | 0 | 0 | 0 | 0 | 0 | 0 | 0 | 0 | 0 |

Beispiel D

Im Gewächshaus wurden die in der Tabelle aufgeführten Verbindungen mit den ebenfalls erwähnten Aufwandmengen appliziert. Hierzu wurden die Wirkstoffe in Gefäße mit 1500 ml Wasser gegeben. Als Testpflanzen wurden Echinochloa crus-galli, Fimbristylis miliacea, Paspalum distichum und Cyperus difformis im 2-bis 5-Blatt-Stadium eingesetzt.

In der folgenden Tabelle bedeuten:

0 = keine Schädigung

1 = schwache Schädigung
2 = mittlere Schädigung
3 = starke Schädigung
4 = total vernichtet
Ec = Echinochloa crus-galli
Fi = Fimbritylis miliacea
Pa = Paspalum distichum
Cy = Cyperus difformis

| Erfindungsgemäße Verbindungen | Wasserapplikation ppm | Ec | Fi | Pa | Cy |
|---|---|---|---|---|---|
| Beispiel 2 | 30 | 4 | 4 | 4 | 4 |
| Beispiel 3 | 30 | 4 | 4 | 4 | 4 |

Wie die Tabelle zeigt, sind die erfindungsgemäßen Verbindungen stark wirksam gegen wichtige Reisunkräuter.

**Patentansprüche**

**1.** 6,7-Dihydro-[1,2,4]triazolo[1,5-a][1,3,5]triazin-2-sulfonsäureamide der allgemeinen Formel I

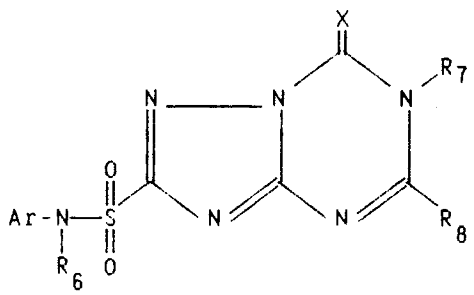

(I) ,

in der

Ar eine Phenylgruppe der allgemeinen Formel

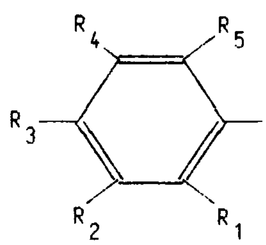

,

$R_1$, $R_2$, $R_3$, $R_4$ und $R_5$, die gleich oder verschieden sind, ein Wasserstoffatom, einen gegebenenfalls ein- oder mehrfach, gleich oder verschieden durch Halogen, Hydroxy, Halogen-$C_1$-$C_4$-alkyl, $C_1$-$C_4$-Alkoxy, Halogen-$C_1$-$C_4$-alkoxy, $C_1$-$C_4$-Alkylthio, $C_1$-$C_4$-Alkylsulfinyl oder $C_1$-$C_4$-Alkylsulfonyl substituierten $C_1$-$C_6$-Alkyl-, $C_2$-$C_6$-Alkenyl- oder $C_2$-$C_6$-Alkinylrest, ein Halogenatom, eine $C_1$-$C_4$-Alkoxygruppe, eine Aminocarbonlylgruppe

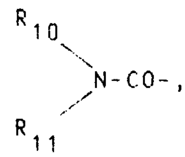

eine Aminogrupppe

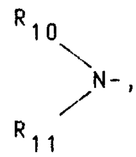

eine Cyanogruppe, eine Nitrogruppe, eine Schwefel enthaltende Gruppe $R_9$-S(O)$_n$-, eine Acylgruppe $R_9$-CO-, eine Gruppe $R_9$-O-CO-($CH_2$)$_n$- oder einen durch $C_1$-$C_4$-Alkyl, Halogen oder Nitro substituierten Phenyl- oder Phenoxyrest,

$R_6$ ein Wasserstoffatom, eine Acylgruppe $R_9$-CO-, eine Gruppe $R_9$-O-CO-, eine $C_1$-$C_6$-Alkylgruppe, eine $C_2$-$C_6$-Alkenylgruppe, eine $C_2$-$C_6$-Alkinylgruppe, eine Phenyl-$C_1$-$C_4$-alkylgruppe, eine Aminocarbonylgruppe

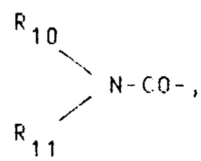

ein Alkalimetallatom, ein einwertiges Metalläquivalent aus der Gruppe der Erdalkalimetalle und weiterer Metalle oder einen gegebenenfalls durch $C_1$-$C_6$-Alkyl substituierten Ammoniumrest,

$R_7$ und $R_8$, die gleich oder verschieden sind, ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl oder Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest, einen durch $R_1$, $R_2$, $R_3$, $R_4$ und $R_5$ substituierten Phenylrest, eine Acylgruppe $R_9$-CO-, eine Gruppe $R_9$-O-CO-($CH_2$)$_n$-, eine Aminocarbonylgruppe

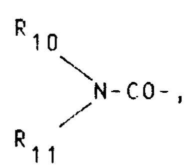

oder eine Sulfonylgruppe $R_9$-$SO_2$-,

$R_9$ ein Wasserstoffatom, eine gegebenenfalls ein- oder mehrfach, gleich oder verschieden durch Halogen, Hydroxy oder $C_1$-$C_4$-Alkoxy substituierte $C_1$-$C_6$-Alkyl-, $C_2$-$C_6$-Alkenyl-, $C_2$-$C_6$-Alkinyl- oder Phenyl-$C_1$-$C_4$-alkylgruppe oder eine gegebenenfalls durch Halogen, Nitro oder $C_1$-$C_4$-Alkyl substituierte Phenylgruppe,

$R_{10}$ und $R_{11}$, die gleich oder verschieden sind, ein Wasserstoffatom, einen gegebenenfalls ein- oder mehrfach, gleich oder verschieden durch Halogen, Hydroxy

oder $C_1$-$C_4$-Alkoxy substituierten $C_1$-$C_6$-Alkyl-, $C_2$-$C_6$-Alkenyl-oder $C_2$-$C_6$-Alkinylrest oder

$R_{10}$ und $R_{11}$ gemeinsam mit dem benachbarten Stickstoffatom die Pyrrolidinyl-, Piperidino- oder Morpholinogruppe,

X ein Sauerstoff- oder Schwefelatom und

n 0, 1 oder 2

bedeuten.

**2.** 6,7-Dihydro-[1,2,4]triazolo[1,5-a][1,3,5]triazin-2-sulfonsäureamide gemäß Anspruch 1, dadurch gekennzeichnet, daß in der allgemeinen Formel I Ar eine Phenylgruppe der allgemeinen Formel

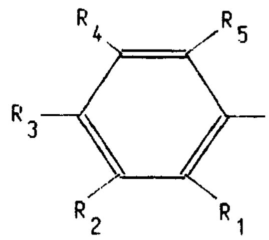

| | |
|---|---|
| $R_1$ und $R_5$, | die gleich oder verschieden sind, ein Halogenatom, eine Methylgruppe, eine Trifluormethylgruppe, eine Nitrogruppe, eine Methoxygruppe oder eine Methoxycarbonylgruppe, |
| $R_2$, $R_3$ und $R_4$, | die gleich oder verschieden sind, ein Wasserstoffatom, ein Halogenatom, eine Trifluormethyl- oder eine $C_1$-$C_4$-Alkylgruppe, |
| $R_6$ | ein Wasserstoffatom, ein einwertiges Metalläquivalent oder eine $C_1$-$C_4$-Acylgruppe, |
| $R_7$ und $R_8$, | die gleich oder verschieden sind, ein Wasserstoffatom, eine $C_1$-$C_4$-Acylgruppe, eine $C_1$-$C_4$-Alkylgruppe, eine $C_2$-$C_4$-Alkenylgruppe oder eine Phenylgruppe und |
| X | ein Sauerstoff- oder Schwefelatom |

bedeuten.

**3.** Verfahren zur Herstellung von 6,7-Dihydro-[1,2,4]triazolo[1,5-a][1,3,5]triazin-2-sulfonsäureamiden der allgemeinen Formel I gemäß Anspruch 1, dadurch gekennzeichnet, daß man

A) Amine der allgemeinen Formel II,

$Ar\text{-}NH\text{-}R_6$ (II) ,

in der Ar die oben angegebene Bedeutung hat und $R_6$ ein Wasserstoffatom, eine $C_1$-$C_6$-Alkyl-, $C_2$-$C_6$-Alkenyl- oder $C_2$-$C_6$-Alkinylgruppe bedeutet, mit einem Sulfonsäurechlorid der allgemeinen Formel III

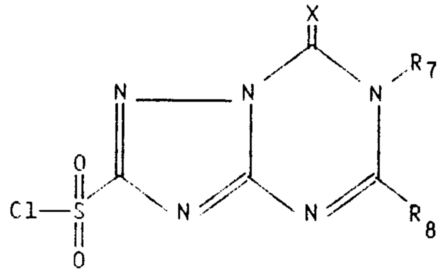

(III) ,

in der $R_7$, $R_8$ und X die oben angegebenen Bedeutungen haben, in einem geeigneten Lösungsmittel in Gegenwart eines Säureakzeptors umsetzt, oder

B) eine Verbindung der allgemeinen Formel IX

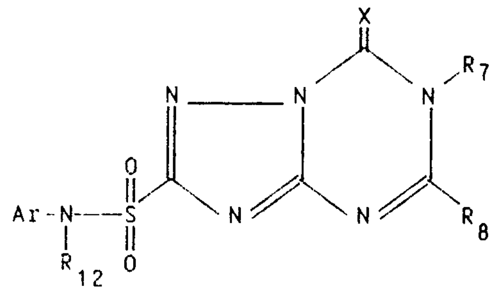

(IX),

in der Ar, $R_7$, $R_8$ und X die oben angegebenen Bedeutungen haben und $R_{12}$ ein Wasserstoffatom oder ein einwertiges Metalläquivalent bedeutet, mit Verbindungen der allgemeinen Formel X

$R_6$-Hal (X) ,

in der $R_6$ die oben genannte Bedeutung hat, aber nicht für ein Wasserstoffatom steht, und Hal, Chlor oder Brom bedeutet, oder der allgemeinen Formel XI

$R_9$-CO-Hal (XI),

in der $R_9$ die oben genannte Bedeutung hat, aber nicht für ein Wasserstoffatom steht, und Hal Chlor oder Brom bedeutet, oder der allgemeinen Formel XII

$R_9$-CO-O-CO-$R_9$ (XII),

in der $R_9$ die oben genannte Bedeutung hat, in einem geeigneten Lösungsmittel umsetzt, oder
C) eine Verbindung der allgemeinen Formel XIII

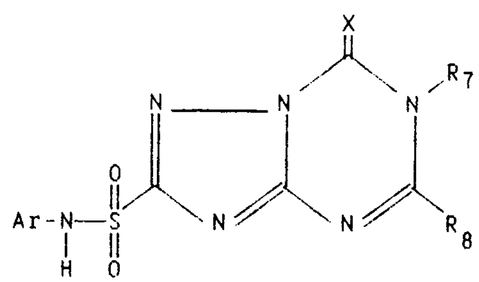

(XIII) ,

in der Ar, $R_7$, $R_8$ und X die oben angegebenen Bedeutungen haben, mit einer Verbindung der allgemeinen Formel XIV

M - Y (XIV),

in der M ein einwertiges Metalläquivalent und Y ein Wasserstoffatom, eine Hydroxy-, Niederalkyl-, Niederalkoxy- oder Aminogruppe bedeutet, in einem Lösungsmittel umsetzt.

**4.** Verfahren zur Herstellung von 6,7-Dihydro-[1,2,4]triazolo[1,5-a][1,3,5]triazin-2-sulfonsäureamiden der allgemeinen Formel I gemäß Anspruch 1, dadurch gekennzeichnet, daß man

D) eine Verbindung der allgemeinen Formel IV

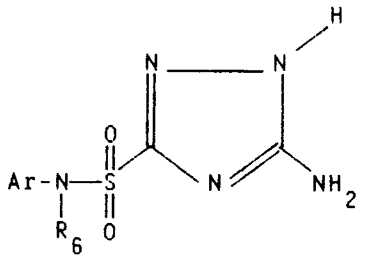

(IV) ,

in der Ar die oben angegebene Bedeutung hat und $R_6$ ein Wasserstoffatom oder eine $C_1$-$C_6$-Alkyl-, $C_2$-$C_6$-Alkenyl- oder $C_2$-$C_6$-Alkinylgruppe bedeutet, mit einem Isocyanat oder Isothiocyanat der Formel V

$R_7$-NCX (V) ,

in der $R_7$ und X die oben angegebenen Bedeutungen haben, in einem geeigneten Lösungsmittel gegebenenfalls in Gegenwart eines Säureakzeptors beziehungsweise Katalysators umsetzt, und die entstandenen Verbindungen der allgemeinen Formel VI

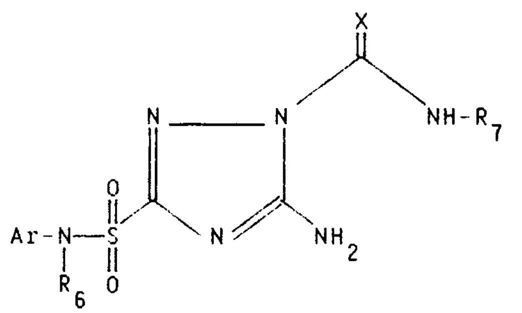

(VI) ,

in der Ar, $R_7$ und X die oben angegebenen Bedeutungen haben und $R_6$ ein Wasserstoffatom, eine $C_1$-$C_6$-Alkyl-, $C_2$-$C_6$-Alkenyl- oder $C_2$-$C_6$-Alkinylgruppe bedeutet, mit einem Orthoester der Formel VII

$R_8$-C($OR_9$)$_3$ (VII) ,

in der $R_9$ die oben angegebene Bedeutung hat, aber nicht für ein Wasserstoffatom steht, und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest oder einen durch $R_1$, $R_2$, $R_3$, $R_4$ und $R_5$ substituierten Phenylrest bedeutet, in einem geeigneten Lösungsmittel, das auch der Orthoester selbst sein kann, umsetzt, oder

E) eine Verbindung der allgemeinen Formel IV

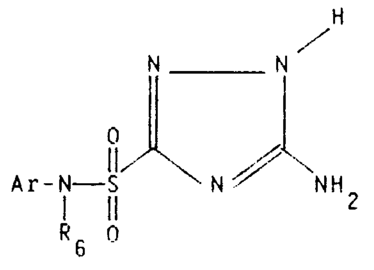

(IV) ,

in der Ar die oben angegebene Bedeutung hat und $R_6$ ein Wasserstoffatom oder eine $C_1$-$C_6$-Alkyl-, $C_2$-$C_6$-Alkenyl- oder $C_2$-$C_6$-Alkinylgruppe bedeutet, mit einem Orthoester der allgemeinen Formel VII

$R_8$-C(O$R_9$)$_3$ (VII) ,

in der $R_9$ die oben angegebene Bedeutung hat, aber nicht für ein Wasserstoffatom steht, und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest oder einen durch $R_1$, $R_2$, $R_3$, $R_4$ und $R_5$ substituierten Phenylrest bedeutet, zu Verbindungen der allgemeinen Formel VIII

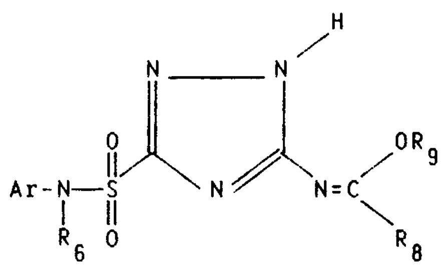

(VIII) ,

in der Ar und $R_9$ die oben angegebenen Bedeutungen haben, $R_9$ aber nicht für ein Wasserstoffatom steht, $R_6$ ein Wasserstoffatom oder eine $C_1$-$C_6$-Alkyl-, $C_2$-$C_6$-Alkenyl- oder $C_2$-$C_6$-Alkinylgruppe und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl-oder Phenyl-$C_2$-$C_6$-alkinylrest oder einen durch $R_1$, $R_2$, $R_3$, $R_4$ und $R_5$ substituierten Phenylrest bedeuten, in einem geeigneten Lösungsmittel, das auch der Orthoester selbst sein kann, umsetzt und dann die Verbindungen der allgemeinen Formel VIII mit einem Isocyanat oder Isothiocyanat der Formel V

$R_7$-NCX (V)

in der $R_7$ und X die oben angegebenen Bedeutungen haben, in einem geeigneten Lösungsmittel gegebenenfalls in Gegenwart eines Säureakzeptors beziehungsweise Katalysators umsetzt.

**5.** Mittel mit herbizider und wachstumsregulierender Wirkung, gekennzeichnet durch einen Gehalt an mindestens einer Verbindung gemäß den Ansprüchen 1 und 2.

**6.** Verwendung von Verbindungen gemäß den Ansprüchen 1 und 2 zur Bekämpfung monokotyler und dikotyler Unkrautarten in landwirtschaftlichen Hauptkulturen.

7. Verfahren zur Herstellung von Mitteln mit herbizider und wachstumsregulie render Wirkung, dadurch gekennzeichnet, daß man Verbindungen der allgemeinen Formel I gemäß den Ansprüchen 1 und 2 mit Träger- und/oder Hilfsstoffen vermischt.

## Claims

1. 6,7-Dihydro-[1,2,4]triazolo[1,5-a][1,3,5]triazine-2-sulphonamides of general formula I

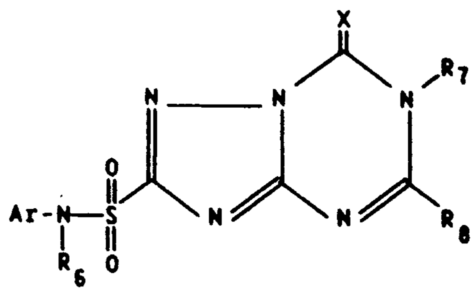

(I),

in which

Ar is a phenyl group of general formula

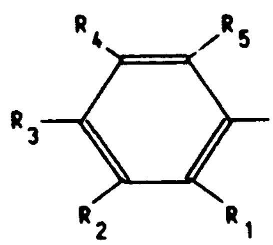

$R_1$, $R_2$, $R_3$, $R_4$ and $R_5$ are the same or different and are a hydrogen atom, a $C_1$-$C_6$-alkyl, $C_2$-$C_6$-alkenyl or $C_2$-$C_6$-alkynyl group, each of which is optionally substituted by one more of the same or different halo, hydroxy, halo-$C_1$-$C_4$-alkyl, $C_1$-$C_4$-alkoxy, halo-$C_1$-$C_4$-alkoxy, $C_1$-$C_4$-alkylthio, $C_1$-$C_4$-alkylsulphinyl or $C_1$-$C_4$-alkylsulphonyl, a halogen atom, a $C_1$-$C_4$-alkoxy group, a carbamoyl group

$R_{10}$\
N-CO-,\
/\
$R_{11}$

an amino group

$R_{10}$\
N-,\
/\
$R_{11}$

a cyano group, a nitro group, a sulphur containing group $R_9$-S(O)$_n$-,an acyl group $R_9$-CO-, a group $R_9$-O-CO-$(CH_2)_n$-, or a phenyl or phenoxy group,

both of which are substituted by $C_1$-$C_4$-alkyl, halo or nitro,

$R_6$ is a hydrogen atom, an acyl group $R_9$-CO-, a group $R_9$-O-CO-, a $C_1$-$C_6$-alkyl group, a $C_2$-$C_6$-alkenyl group, a $C_2$-$C_6$-alkynyl group, a phenyl-$C_1$-$C_4$-alkyl group, a carbamoyl group

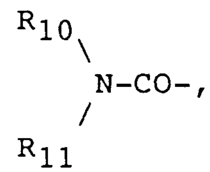

an alkali metal atom, a single metal equivalent of an alkaline earth or other metal, or an ammonium group, optionally substituted by $C_1$-$C_6$-alkyl,

$R_7$ and $R_8$ are the same or different and are a hydrogen atom, a $C_1$-$C_6$-alkyl, $C_2$-$C_6$-alkenyl or $C_2$-$C_6$-alkynyl group, each of which is optionally substituted by halo and / or $C_1$-$C_4$-alkoxy, a phenyl-$C_1$-$C_6$-alkyl, phenyl-$C_2$-$C_6$-alkenyl or phenyl-$C_2$-$C_6$-alkynyl group, each of which is optionally substituted by halo, $C_1$-$C_4$-alkyl, $C_1$-$C_4$-alkoxy, $C_1$-$C_4$-alkoxy-$C_1$-$C_4$-alkyl or halo-$C_1$-$C_4$-alkyl, a phenyl group, substituted by $R_1$, $R_2$, $R_3$, $R_4$ and $R_5$, an acyl group $R_9$-CO-, a group $R_9$-O-CO-$(CH_2)_n$-, a carbamoyl group

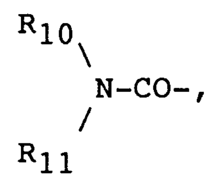

or a sulphonyl group $R_9$-$SO_2$-,

$R_9$ is a hydrogen atom, a $C_1$-$C_6$-alkyl, $C_2$-$C_6$-alkenyl, $C_2$-$C_6$-alkynyl or phenyl-$C_1$-$C_4$-alkyl group, each of which is optionally substituted by one or more of the same or different halo, hydroxy or $C_1$-$C_4$-alkoxy, or a phenyl group optionally substituted by halo, nitro or $C_1$-$C_4$-alkyl,

$R_{10}$ and $R_{11}$ are the same or different and are a hydrogen atom, a $C_1$-$C_6$-alkyl, $C_2$-$C_6$-alkenyl or $C_2$-$C_6$-alkynyl group, each of which is optionally substituted by one or more of the same or different halo, hydroxy or $C_1$-$C_4$-alkoxy, or $R_{10}$ and $R_{11}$ together with the adjacent nitrogen atom form a pyrrolidinyl, piperidino or morpholino group,

X is an oxygen or sulphur atom, and

n is 0, 1 or 2.

**2.** 6,7-Dihydro-[1,2,4]triazolo[1,5-a][1,3,5]triazine-2-sulphonamidesaccording to claim 1, characterised by that in the general formula I

Ar is a phenyl group of general formula

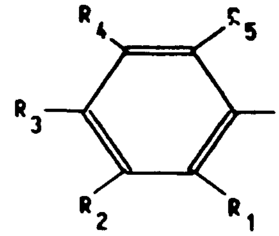

| | |
|---|---|
| $R_1$ and $R_5$ | are the same or different and are a halogen atom, a methyl group, a trifluoromethyl group, a nitro group, a methoxy group or a methoxycarbonyl group, |
| $R_2$, $R_3$ and $R_4$ | are the same or different and are a hydrogen atom, a halogen atom, a trifluoromethyl or a $C_1$-$C_4$-alkyl group, |
| $R_6$ | is a hydrogen atom, a single equivalent of a metal or a $C_1$-$C_4$-acyl group, and |
| $R_7$ and $R_8$ | are the same or different and are a hydrogen atom, a $C_1$-$C_4$-acyl group, a $C_1$-$C_4$-alkyl group, a $C_2$-$C_4$-alkenyl group or a phenyl group and |
| X | is an oxygen or sulphur atom. |

**3.** Process for the preparation of 6,7-dihydro-[1,2,4]triazolo[1,5-a][1,3,5]triazine-2-sulphonamides of general formula I, according to claim 1, characterised by

A) reacting an amine of general formula II,

Ar - NH - $R_6$ (II),

in which Ar has the meaning given above and $R_6$ is a hydrogen atom, a $C_1$-$C_6$-alkyl, $C_2$-$C_6$-alkenyl or $C_2$-$C_6$-alkynyl group, with a sulphonyl chloride of general formula III

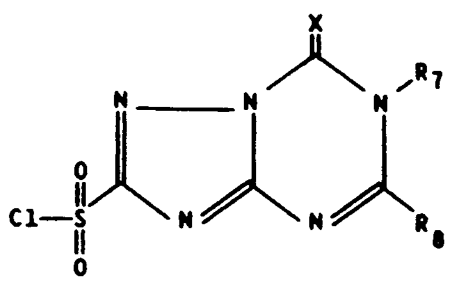

(III),

in which $R_7$, $R_8$ and X have the meanings given above in a suitable solvent and in the presence of an acid acceptor, or

B) reacting a compound of general formula IX

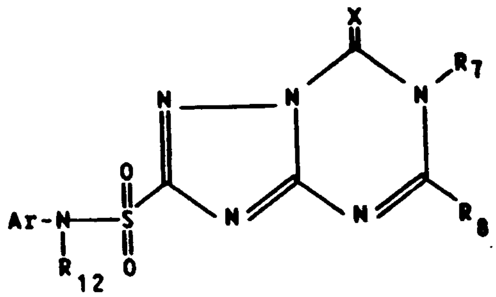

(IX),

in which Ar, $R_7$, $R_8$ and X have the meanings given above and $R_{12}$ is a hydrogen atom or a single equivalent of a metal, with a compound of general fomula X

$R_6$ - Hal (X),

in which $R_6$ has the meaning given above, except a hydrogen atom and Hal is chlorine or bromine, or of general formula XI

$R_9$ - Co - Hal (XI),

in which $R_9$ has the meaning given above, except a hydrogen atom and Hal is chlorine or bromine, or of general formula XII

$R_9$ - CO - O - $COR_9$ (XII),

in which $R_9$ has the meaning given above, in a suitable solvent, or
C) reacting a compound of genral formula XIII

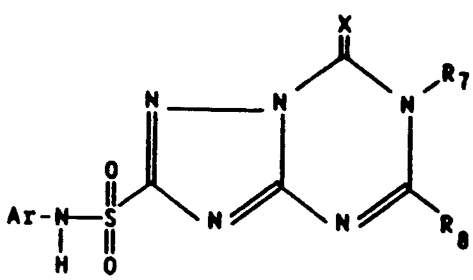

(XIII),

in which Ar, $R_7$, $R_8$ and X have the meanings given above, with a compound of general formula XIV

M - Y (XIV),

in which M is a single equivalent of a metal, and Y is a hydrogen atom, a hydroxy, lower alkyl, lower alkoxy or an amino group, in a solvent.

**4.** Process for the preparation of 6,7-dihydro-[1,2,4]triazolo[1,5-a][1,3,5]triazine-2-sulphonamides of general formula I, according to claim 1, characterised by

D) reacting a compound general formula IV

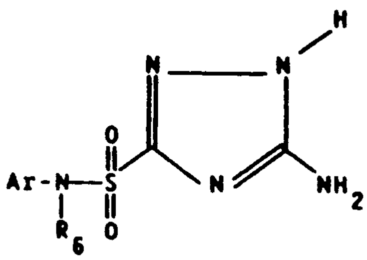

(IV),

in which Ar has the meaning given above and $R_6$ is a hydrogen atom, a $C_1$-$C_6$-alkyl, $C_2$-$C_6$-alkenyl or $C_2$-$C_6$-alkynyl group, an isocyanate or isothiocyanate of formula V

$R_7$ - NCX (V),

in which $R_7$ and X have the meanings given above in a suitable solvent, optionally in the presence of an acid acceptor and / or catalyst, and reacting the resulting compound of general formula VI

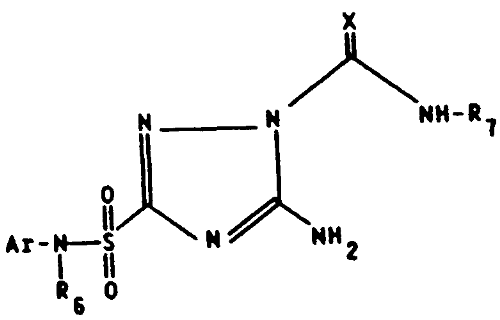

(VI),

in which Ar, $R_7$ and X have the meanings given above and $R_6$ is a hydrogen atom, a $C_1$-$C_6$-alkyl, $C_2$-$C_6$-alkenyl or $C_2$-$C_6$-alkynyl group, with an orthoester of formula VII

$R_8$ - C $(OR_9)_3$ (VII),

in which, $R_9$ has the meaning given above, except a hydrogen atom and $R_8$ is a hydrogen atom, a $C_1$-$C_6$-alkyl, $C_2$-$C_6$-alkenyl or $C_2$-$C_6$-alkynyl group, each of which is optionally substituted by halo and / or $C_1$-$C_4$-alkoxy, a phenyl-$C_1$-$C_6$-alkyl, phenyl-$C_2$-$C_6$-alkenyl or phenyl-$C_2$-$C_6$-alkynyl group, each of which is optionally substituted by halo, $C_1$-$C_4$-alkyl, $C_1$-$C_4$-alkoxy, $C_1$-$C_4$-alkoxy-$C_1$-$C_4$-alkyl or halo-$C_1$-$C_4$-alkyl, or phenyl, substituted by $R_1$, $R_2$, $R_3$, $R_4$ and $R_5$, in a suitable solvent, which can be the orthoester itself, or

E) reacting a compound of general formula IV

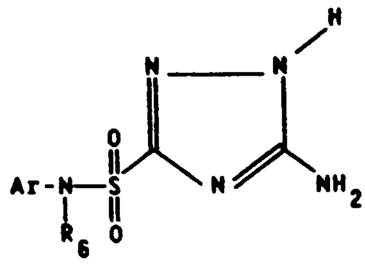

(IV),

in which Ar has the meaning given above and $R_6$ is a hydrogen atom, a $C_1$-$C_6$-alkyl, $C_2$-$C_6$-alkenyl or $C_2$-$C_6$-alkynyl group, with an orthoester of general formula VII

$R_8$ - C $(OR_9)_3$ (VII),

in which $R_9$ has the meaning given above, except a hydrogen atom, and $R_8$ is a hydrogen atom, a $C_1$-$C_6$-alkyl, $C_2$-$C_6$-alkenyl or $C_2$-$C_6$-alkynyl group, each of which is optionally substituted by halo and / or $C_1$-$C_4$-alkoxy, a phenyl-$C_1$-$C_6$-alkyl, phenyl-$C_2$-$C_6$-alkenyl or phenyl-$C_2$-$C_6$-alkynyl group, each of which is optionally substituted by halo, $C_1$-$C_4$-alkyl, $C_1$-$C_4$-alkoxy, $C_1$-$C_4$-alkoxy-$C_1$-$C_4$-alkyl or halo-$C_1$-$C_4$-alkyl, or a phenyl group, substituted by $R_1$, $R_2$, $R_3$, $R_4$ and $R_5$, to compounds of general formula VIII

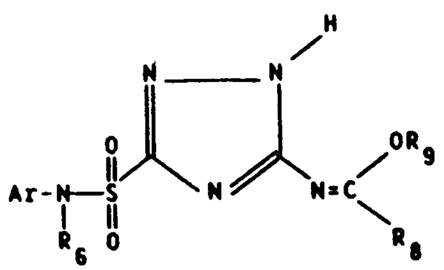

(VIII),

in which Ar and $R_9$ have the meanings given above, except a hydrogen atom, $R_6$ is a hydrogen atom, a $C_1$-$C_6$-alkyl, $C_2$-$C_6$-alkenyl or $C_2$-$C_6$-alkynyl group, and $R_8$ is a hydrogen atom, a $C_1$-$C_6$-alkyl, $C_2$-$C_6$-alkenyl or $C_2$-$C_6$-alkynyl group, each of which is optionally substituted by halo and / or $C_1$-$C_4$-alkoxy, a phenyl-$C_1$-$C_6$-alkyl, phenyl-$C_2$-$C_6$-alkenyl or phenyl-$C_2$-$C_6$-alkynyl group, each of which is optionally substituted by halo, $C_1$-$C_4$-alkyl, $C_1$-$C_4$-alkoxy, $C_1$-$C_4$-alkoxy-$C_1$-$C_4$-alkyl or halo-$C_1$-$C_4$-alkyl, or a phenyl group, substituted by $R_1$, $R_2$, $R_3$, $R_4$ and $R_5$, in a suitable solvent, which can be the orthoester itself, and reacting the resulting compound of general formula VIII, with an isocyanate or isothiocyanate of formula V

$R_7$ - NCX (V),

in which $R_7$ and X have the meanings given above in a suitable solvent, optionally in the presence of an acid acceptor and / or catalyst.

**5.** A herbicidal and plant-growth regulant composition characterised by a content of at least one compound according to claim 1 and 2.

**6.** Use of compounds according to claim 1 and 2 for combatting monocotyledonous and dicotyledonous weeds in agricultural main cultures.

7. Process for the preparation of compositions, having herbicidal and plant growth regulatory activity, characterised by mixing compounds of general formula I, according to claim 1 and 2, with carriers and / or diluents.

## Revendications

1. 6,7-Dihydro-[1,2,4]triazolo[1,5-a][1,3,5]triazine-2-sulfonamides répondant à la formule générale I :

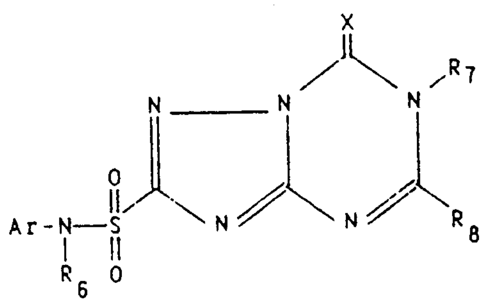

(I) ,

dans laquelle

Ar représente un radical phényle de formule générale :

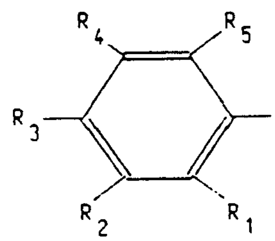

,

$R_1$, $R_2$, $R_3$, $R_4$ et $R_5$, qui sont identiques ou différents, représentent chacun un atome d'hydrogène, un radical $C_1$-$C_6$-alkyle, $C_2$-$C_6$-alcényle ou $C_2$-$C_6$-alcynyle éventuellement porteur d'un ou de plusieurs substituants, identiques ou différents, pris dans l'ensemble constitué par les halogènes et les radicaux hydroxy, halogéno-$C_1$-$C_4$-alkyles, $C_1$-$C_4$-alcoxy, halogéno-$C_1$-$C_4$-alcoxy, $C_1$-$C_4$-alkylthio, $C_1$-$C_4$-alkylsulfinyles et $C_1$-$C_4$-alkylsulfonyles, un atome d'halogène, un $C_1$-$C_4$-alcoxy, un aminocarbonyle

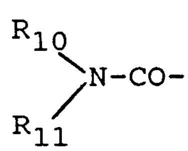

,

un amino

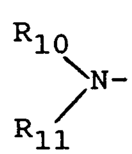

,

un cyano, un nitro, un radical sulfuré $R_9$-S(O)$_n$-, un acyle $R_9$-CO-, un radical $R_9$-O-CO-$(CH_2)_n$- ou un radical phényle ou phénoxy porteur d'un $C_1$-$C_4$-alkyle, d'un halogène ou d'un nitro,

$R_6$ représente un atome d'hydrogène, un acyle $R_9$-CO-, un radical $R_9$-O-CO-,

un $C_1$-$C_6$-alkyle, un $C_2$-$C_6$-alcényle, un $C_2$-$C_6$-alcynyle, un phényl-$C_1$-$C_4$-alkyle, un aminocarbonyle

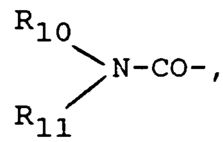

un atome de métal alcalin, un équivalent univalent d'un métal du groupe des métaux alcalino-terreux et d'autres métaux, ou un radical ammonium éventuellement porteur d'un alkyle en $C_1$-$C_6$,

$R_7$ et $R_8$, qui sont identiques ou différents, représentent chacun un atome d'hydrogène, un radical $C_1$-$C_6$-alkyle, $C_2$-$C_6$-alcényle ou $C_2$-$C_6$-alcynyle éventuellement porteur d'un halogène et/ou d'un $C_1$-$C_4$-alcoxy, un radical phényl-$C_1$-$C_6$-alkyle, phényl-$C_2$-$C_6$-alcényle ou phényl-$C_2$-$C_6$-alcynyle éventuellement porteur d'un halogène, d'un $C_1$-$C_4$-alkyle, d'un $C_1$-$C_4$-alcoxy, d'un $C_1$-$C_4$-alcoxy-$C_1$-$C_4$-alkyle ou d'un halogéno-$C_1$-$C_4$-alkyle, un phényle porteur de substituants $R_1$, $R_2$, $R_3$, $R_4$ et $R_5$, un acyle $R_9$-CO-, un radical $R_9$-O-CO-$(CH_2)_n$-, un aminocarbonyle

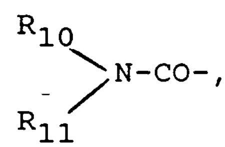

ou un radical sulfonyle $R_9$-$SO_2$-,

$R_9$ représente un atome d'hydrogène, un radical $C_1$-$C_6$-alkyle, $C_2$-$C_6$-alcényle, $C_2$-$C_6$-alcynyle ou phényl-$C_1$-$C_4$-alkyle éventuellement porteur d'un ou de plusieurs substituants, identiques ou différents, pris dans l'ensemble constitué par les halogènes et les radicaux hydroxy et $C_1$-$C_4$-alcoxy, ou un phényle éventuellement porteur d'un halogène, d'un nitro ou d'un $C_1$-$C_4$-alkyle,

$R_{10}$ et $R_{11}$, qui sont identiques ou différents, représentent chacun un atome d'hydrogène, ou un radical $C_1$-$C_6$-alkyle, $C_2$-$C_6$-alcényle ou $C_2$-$C_6$-alcynyle éventuellement porteur d'un ou de plusieurs substituants, identiques ou différents, pris dans l'ensemble constitué par les halogènes et les radicaux hydroxy et $C_1$-$C_4$-alcoxy, ou

$R_{10}$ et $R_{11}$ forment ensemble, et avec l'atome d'azote voisin, un radical pyrrolidinyle, pipéridino ou morpholino,

X représente un atome d'oxygène ou de soufre et

n est égal à 0, à 1 ou à 2.

**2.** 6,7-Dihydro-[1,2,4]triazolo[1,5-a][1,3,5]triazine-2-sulfonamides selon la revendication 1, caractérisés en ce que, dans la formule générale I :

Ar représente un radical phényle de formule générale

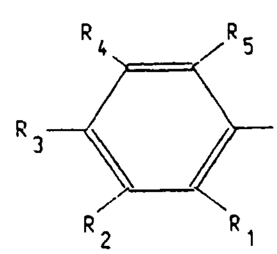

| | |
|---|---|
| $R_1$ et $R_5$, | qui sont identiques ou différents, représentent chacun un atome d'halogène ou un radical méthyle, trifluorométhyle, nitro, méthoxy ou méthoxycarbonyle, |
| $R_2$, $R_3$ et $R_4$, | qui sont identiques ou différents, représentent chacun un atome d'hydrogène ou d'halogène ou un radical trifluorométhyle ou $C_1$-$C_4$-alkyle, |
| $R_6$ | représente un atome d'hydrogène, un équivalent univalent d'un métal ou un radical $C_1$-$C_4$-acyle, |
| $R_7$ et $R_8$, | qui sont identiques ou différents, représentent chacun un atome d'hydrogène ou un radical $C_1$-$C_4$-acyle,$C_1$-$C_4$-alkyle, $C_2$-$C_4$-alcényle ou phényle, et |
| X | représente un atome d'oxygène ou de soufre. |

**3.** Procédé pour préparer des 6,7-dihydro-[1,2,4]triazolo[1,5-a][1,3,5]triazine-2-sulfonamides de formule générale I selon la revendication 1, procédé caractérisé en ce que :

A) on fait réagir des amines répondant à la formule générale II :

Ar-NH-$R_6$ (II)

dans laquelle Ar a la signification qui lui a été donnée ci-dessus et $R_6$ représente un atome d'hydrogène ou un radical $C_1$-$C_6$-alkyle, $C_2$-$C_6$-alcényle ou $C_2$-$C_6$-alcynyle, avec un chlorure d'acide sulfonique répondant à la formule générale III :

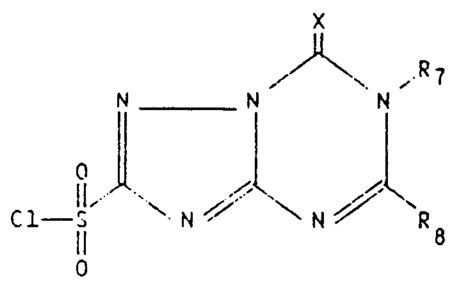

(III) ,

dans laquelle $R_7$, $R_8$ et X ont les significations qui leur ont été données ci-dessus, dans un solvant approprié, en présence d'un accepteur d'acides, ou

B) on fait réagir, dans un solvant approprié, un composé répondant à la formule générale IX

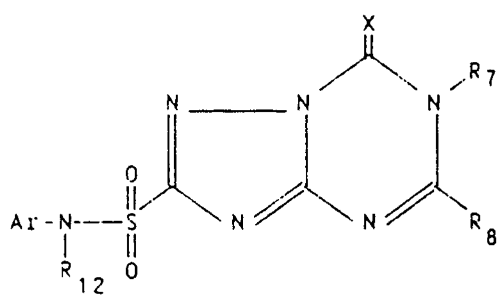

(IX),

dans laquelle Ar, $R_7$, $R_8$ et X ont les significations qui leur ont été données ci-dessus, et $R_{12}$ représente un atome d'hydrogène ou un équivalent univalent d'un métal, avec des composés répondant à la formule générale X :

$R_6$-Hal (X)

dans laquelle $R_6$ a la signification indiquée ci-dessus mais ne représente pas un atome d'hydrogène, et Hal représente le chlore ou le brome, ou répondant à la formule générale XI :

$R_9$-CO-Hal (XI)

dans laquelle $R_9$ a la signification indiquée ci-dessus mais ne représente pas un atome d'hydrogène,

et Hal représente le chlore ou le brome, ou répondant à la formule générale XII :

$R_9$-CO-O-CO-$R_9$ (XII)

dans laquelle $R_9$ a la signification indiquée ci-dessus, ou
C) on fait réagir, dans un solvant, un composé répondant à la formule générale XIII

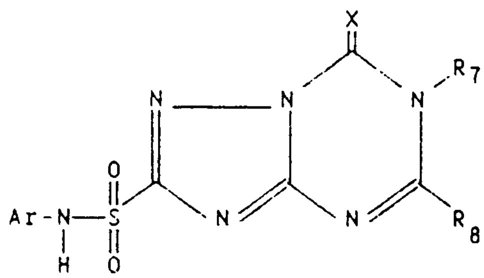

(XIII) ,

dans laquelle Ar, $R_7$, $R_8$ et X ont les significations qui leur ont été données ci-dessus, avec un composé répondant à la formule générale XIV

M - Y (XIV)

dans laquelle M représente un équivalent univalent d'un métal et Y un atome d'hydrogène, un hydroxy, un alkyle inférieur, un alcoxy inférieur ou un amino.

**4.** Procédé pour préparer des 6,7-dihydro-[1,2,4]triazolo[1,5-a][1,3,5]triazine-2-sulfonamides de formule générale I selon la revendication 1, procédé caractérisé en ce que :
D) on fait réagir un composé répondant à la formule générale IV :

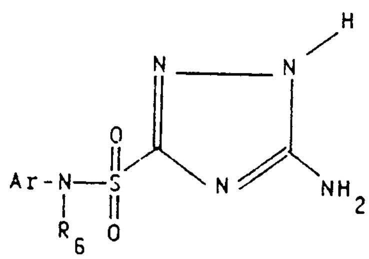

(IV) ,

dans laquelle Ar a la signification indiquée ci-dessus et $R_6$ représente un atome d'hydrogène ou un radical $C_1$-$C_6$-alkyle, $C_2$-$C_6$-alcényle ou $C_2$-$C_6$-alcynyle, avec un isocyanate ou un isothiocyanate répondant à la formule générale V :

$R_7$-NCX (V)

dans laquelle $R_7$ et X ont les significations qui leur ont été données ci-dessus, dans un solvant approprié, éventuellement en présence d'un accepteur d'acides, ou d'un catalyseur, et on fait réagir les composés formés, qui répondent à la formule générale VI :

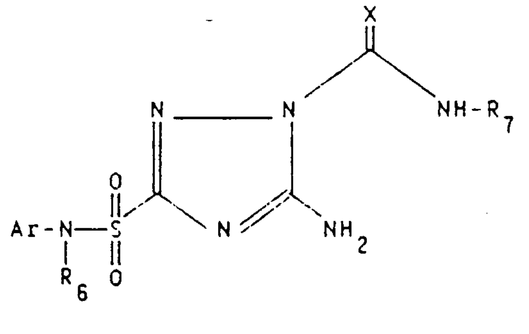

(VI) ,

dans laquelle Ar, $R_7$ et X ont les significations qui leur ont été données ci-dessus et $R_6$ représente un atome d'hydrogène ou un radical $C_1$-$C_6$-alkyle, $C_2$-$C_6$-alcényle ou $C_2$-$C_6$-alcynyle, avec un ortho-ester répondant à la formule VII :

$R_8$-$C(OR_9)_3$ (VII)

dans laquelle $R_9$ a la signification indiquée ci-dessus mais ne représente pas un atome d'hydrogène, et $R_8$ représente un atome d'hydrogène, un radical $C_1$-$C_6$-alkyle, $C_2$-$C_6$-alcényle ou $C_2$-$C_6$-alcynyle éventuellement porteur d'un halogène et/ou d'un $C_1$-$C_4$-alcoxy, un radical phényl-$C_1$-$C_6$-alkyle, phényl-$C_2$-$C_6$-alcényle ou phényl-$C_2$-$C_6$-alcynyle éventuellement porteur d'un halogène, d'un $C_1$-$C_4$-alkyle, d'un $C_1$-$C_4$-alcoxy, d'un $C_1$-$C_4$-alcoxy-$C_1$-$C_4$-alkyle ou d'un halogéno-$C_1$-$C_4$-alkyle, ou un radical phényle porteur de substituants $R_1$, $R_2$, $R_3$, $R_4$ et $R_5$, dans un solvant approprié, qui peut également être l'ortho-ester lui-même, ou

E) on fait réagir un composé répondant à la formule générale IV :

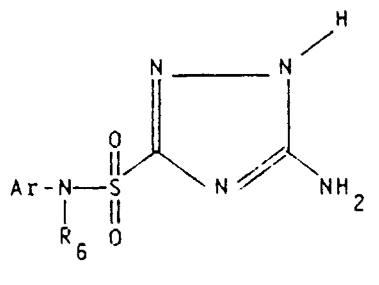

(IV) ,

dans laquelle Ar a la signification indiquée ci-dessus et $R_6$ représente un atome d'hydrogène ou un radical $C_1$-$C_6$-alkyle, $C_2$-$C_6$-alcényle ou $C_2$-$C_6$-alcynyle, avec un orthoester répondant à la formule générale VII :

$R_8$-$C(OR_9)_3$ (VII)

dans laquelle $R_9$ a la signification indiquée ci-dessus mais ne représente pas un atome d'hydrogène, et $R_8$ représente un atome d'hydrogène, un radical $C_1$-$C_6$-alkyle, $C_2$-$C_6$-alcényle ou $C_2$-$C_6$-alcynyle, éventuellement porteur d'un halogène et/ou d'un $C_1$-$C_4$-alcoxy, un radical phényl-$C_1$-$C_6$-alkyle, phényl-$C_2$-$C_6$-alcényle ou phényl-$C_2$-$C_6$-alcynyle éventuellement porteur d'un halogène, d'un $C_1$-$C_4$-alkyle, d'un $C_1$-$C_4$-alcoxy, d'un $C_1$-$C_4$-alcoxy-$C_1$-$C_4$-alkyle ou d'un halogéno-$C_1$-$C_4$-alkyle, ou un radical phényle porteur de substituants $R_1$, $R_2$, $R_3$, $R_4$ et $R_5$, de manière à obtenir des composés répondant à la formule générale VIII :

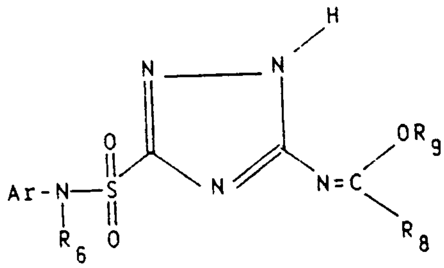

(VIII) ,

dans laquelle Ar et $R_9$ ont les significations qui leur ont été données ci-dessus mais $R_9$ ne représente pas un atome d'hydrogène, $R_6$ représente un atome d'hydrogène ou un radical $C_1$-$C_6$-alkyle, $C_2$-$C_6$-alcényle ou $C_2$-$C_6$-alcynyle et $R_8$ représente un atome d'hydrogène, un radical $C_1$-$C_6$-alkyle, $C_2$-$C_6$-alcényle ou $C_2$-$C_6$-alcynyle éventuellement porteur d'un halogène et/ou d'un $C_1$-$C_4$-alcoxy, un radical phényl-$C_1$-$C_6$-alkyle, phényl-$C_2$-$C_6$-alcényle ou phényl-$C_2$-$C_6$-alcynyle éventuellement porteur d'un halogène, d'un $C_1$-$C_4$-alkyle, d'un $C_1$-$C_4$-alcoxy, d'un $C_1$-$C_4$-alcoxy-$C_1$-$C_4$-alkyle ou d'un halogéno-$C_1$-$C_4$-alkyle, ou un radical phényle porteur de substituants $R_1$, $R_2$, $R_3$, $R_4$ et $R_5$, dans un solvant approprié, qui peut également être l'ortho-ester lui-même, puis on fait réagir les composés de formule générale VIII avec un isocyanate ou un isothiocyanate répondant à la formule V :

$R_7$-NCX (V)

dans laquelle $R_7$ et X ont les significations qui leur ont été données ci-dessus, dans un solvant approprié, éventuellement en présence d'un accepteur d'acides ou d'un catalyseur.

**5.** Produits ayant une action herbicide et régulatrice de croissance, caractérisés en ce qu'ils contiennent au moins un composé selon l'une des revendications 1 et 2.

**6.** Application de composés selon l'une des revendications 1 et 2 à la lutte contre des plantes adventices monocotylédones et dicotylédones dans la grande agriculture.

**7.** Procédé pour préparer des produits ayant une action herbicide et régulatrice de croissance, procédé caractérisé en ce qu'on mélange des composés de formule générale I selon l'une des revendications 1 et 2 avec des supports et/ou des adjuvants.